(19) Europäisches Patentamt
European Patent Office
Office européen des brevets

(11) Veröffentlichungsnummer: **0 257 479**
**A1**

(12) # EUROPÄISCHE PATENTANMELDUNG

(21) Anmeldenummer: **87111791.7**

(22) Anmeldetag: **14.08.87**

(51) Int. Cl.4: **C07D 231/16**, C07D 401/04, A01N 43/56

(30) Priorität: **26.08.86 DE 3628892**

(43) Veröffentlichungstag der Anmeldung:
**02.03.88 Patentblatt 88/09**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jensen-Korte, Uta, Dr.**
**Gelbelstrasse 9**
**D-4000 Düsseldorf 1(DE)**
Erfinder: **Gehring, Reinhold, Dr.**
**Dasnöckelweg 49**
**D-5600 Wuppertal 1(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Becker, Benedikt, Dr.**
**Metzkausener Strasse 14**
**D-4020 Mettmann(DE)**
Erfinder: **Behrenz, Wolfgang, Dr.**
**Untergründemich 14**
**D-5063 Overath(DE)**
Erfinder: **Stendel, Wilhelm, Dr.**
**In den Birken 55**
**D-5600 Wuppertal 1(DE)**

(54) **Substituierte 1-Aryl-3-tert.-butyl-pyrazole.**

(57) Es wurden neue 1-Aryl-3-tert.-butyl-pyrazole der allgemeinen Formel (I),

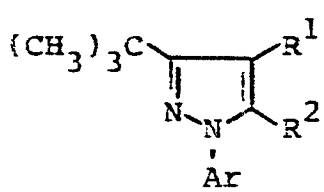

(I)

bereitgestellt,
in welcher
$R^1$ für Nitro oder Halogen steht,
$R^2$ für Halogen oder die Gruppierung $-NR^3R^4$ steht, sowie auch für Wasserstoff steht, wenn $R^1$ Halogen bedeutet, wobei

EP 0 257 479 A1

$R^3$ für Wasserstoff, für jeweils gegebenenfalls substituiertes Alkyl, Alkenyl oder Alkinyl sowie für jeweils gegebenenfalls substituiertes Cycloalkyl oder Cycloalkylalkyl steht und

$R^4$ unabhängig von $R^3$ für die gleichen Reste wie $R^3$ steht, sowie zusätzlich für einen Rest 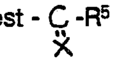

steht, wobei

X für Sauerstoff oder Schwefel steht und

$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und

Ar für gegebenenfalls substituiertes Phenyl, ausgenommen Dinitrophenyl, oder für gegebenenfalls substituiertes Pyridyl steht.

Die neuen Verbindungen der Formel (I) besitzen stark ausgeprägte insektizide und akarizide Eigenschaften.

## Substituierte 1-Aryl-3-tert.-butyl-pyrazole

Die vorliegende Erfindung betrifft neue substituierte 1-Aryl-3-tert.-butyl-pyrazole, mehrere Verfahren zu ihrer Herstellung sowie ihre Verwendung als Schädlingsbekämpfungsmittel, insbesondere als Insektizide und Akarizide.

Es ist bereits bekannt, daß bestimmte in 4-Stellung durch eine Cyano-Gruppe substituierte 5-Amino-1-phenyl-pyrazole, wie beispielsweise das 4-Cyano-5-propionamido-1-(2,4,6-trichlorphenyl)-pyrazol herbizide Eigenschaften besitzen (vgl. z.B. DE-OS 3 226 513).

Außerdem ist bekannt, daß Pyrazole, wie beispielsweise 5-Dimethylaminocarbonyloxy-1-isopropyl-3-methylsulfinylmethylpyrazol oder 1-Cyclohexyl-5-dimethylaminocarbonyloxy-3-methylthiomethyl-pyrazol insektizide Eigenschaften besitzen (vgl. DE-OS 2 819 932 und DE-OS 2 839 270).

Die insektizide Wirkung all dieser Verbindungen ist jedoch, insbesondere bei niedrigen Aufwandmengen oder -kon zentrationen, nicht immer gegenüber allen Schadinsekten voll zufriedenstellend.

Es wurden neue substituierte 1-Aryl-3-tert.-butyl-pyrazole der allgemeinen Formel (I),

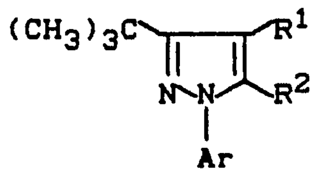

(I)

in welcher

$R^1$ für Nitro oder Halogen steht,

$R^2$ für Halogen oder die Gruppirung $-NR^3R^4$ steht, oder auch für Wasserstoff steht, wenn $R^1$ Halogen bedeutet, wobei

$R^3$ für Wasserstoff, für jeweils gegebenenfalls substituiertes Alkyl, Alkenyl oder Alkinyl oder für jeweils gegebenenfalls substituiertes Cycloalkyl oder Cycloalkylalkyl steht und

$R^4$ unabhängig von $R^3$ für die gleichen Reste wie $R^3$ steht, oder zusätzlich für einen Rest 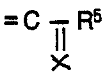

steht, wobei

X für Sauerstoff oder Schwefel steht und

$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und

Ar für gegebenenfalls substituiertes Phenyl, ausgenommen Dinitrophenyl, oder für gegebenenfalls substituiertes Pyridyl steht,

gefunden.

Weiterhin wurde gefunden, daß man die neuen substituierten 1-Aryl-3-tert.-butyl-pyrazole der allgemeinen Formel (I),

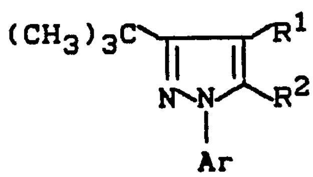

(I)

in welcher

$R^1$ für Nitro oder Halogen steht,

$R^2$ für Halogen oder die Gruppierung $-NR^3R^4$ steht, oder auch für Wasserstoff steht, wenn $R^1$ Halogen bedeutet, wobei

$R^3$ für Wasserstoff, für jeweils gegebenenfalls substituiertes Alkyl, Alkenyl oder Alkinyl oder für jeweils gegebenenfalls substituiertes Cycloalkyl oder Cycloalkylalkyl steht und

$R^4$ unabhängig von $R^3$ für die gleichen Reste wie $R^3$ steht oder zusätzlich für einen Rest 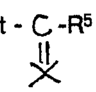

steht, wobei
X für Sauerstoff oder Schwefel steht und
$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und
Ar für gegebenenfalls substituiertes Phenyl, ausgenommen Dinitrophenyl, oder für gegebenenfalls substituiertes Pyridyl steht,
mit Hilfe der im folenden beschriebenen Verfahren erhält:
a) man erhält die substituierten 1-Aryl-3-tert.-butyl-pyrazole der Formel (I),

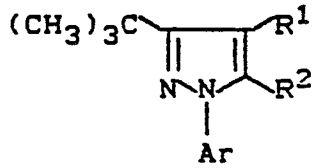

(I)

in welcher
$R^1$, $R^2$ und Ar die oben angegebene Bedeutung haben,
wenn man 1-Aryl-3-tert.-butyl-pyrazole der Formel (II),

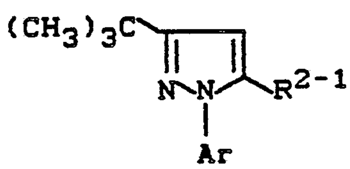

(II)

in welcher
$R^{2-1}$ für Wasserstoff, Halogen oder die Gruppierung $-NR^3R^4$ steht, wobei
$R^3$ und $R^4$ die oben angegbene Bedeutung haben und
Ar die oben angegebene Bedeutung hat,
mit Halogenierungs-oder Nitrierungsmitteln der Formel (III),

$R^1$ -A (III)

in welcher
$R^1$ die oben angegebene Bedeutung hat und
A für eine elektronenanziehende Abgangsgruppe steht,
gegebenenfalls in Gegenwart eines Verdünnungsmittels und gegebenenfalls in Gegenwart eines Katalysators oder Reaktionshilfsmittels umsetzt;
b) man erhält substituierte 1-Aryl-3-tert.-butyl-pyrazole der Formel (Ia),

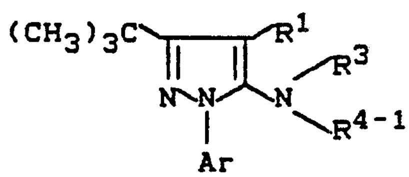

(Ia)

in welcher
$R^1$, $R^3$ und Ar die oben angegebene Bedeutung haben und
$R^{4-1}$ unabhängig von $R^3$ für die gleichen Reste wie $R^3$ steht,
wenn man die nach Verfahren (a) erhältlichen 1-Aryl-5-halogen-3-tert.-butyl-pyrazole der Formel (Ib),

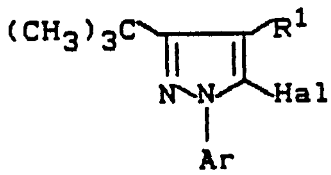

(Ib)

in welcher
$R^1$ und Ar die oben angegebene Bedeutung haben und
Hal für Halogen vorzugsweise für Brom oder Chlor steht,
mit Aminen der Formel (IV),

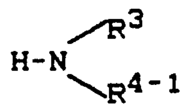

(IV)

in welcher
$R^3$ und $R^{4-1}$ die oben angegebene Bedeutung haben,
gegebenenfalls in Gegenwart eines Verdünnungsmittels und gegebenenfalls' in Gegenwart eines Säurebindemittels umsetzt;
c) man erhält substituierte 1-Aryl-3-tert.-butyl-pyrazole der Formel (Ic),

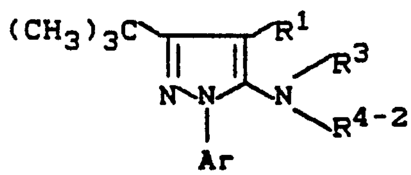

(Ic)

in welcher
$R^1$, $R^3$ und Ar die oben angegebene Bedeutung haben und
$R^{4-1}$ für die oben angegebenen Bedeutungen von $R^4$, ausgenommen Wasserstoff, steht,
wenn man die nach den Verfahren (a) und (b) erhältlichen 5-Amino-1-aryl-3-tert.-butyl-pyrazole der Formel (Id),

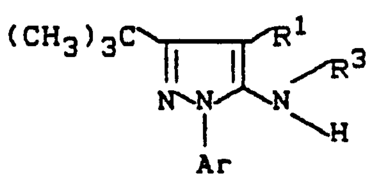

(Id)

in welcher
$R^1$, $R^3$ und Ar die oben angegebene Bedeutung haben,
α) mit Acylierungsmitteln der Formel (V),

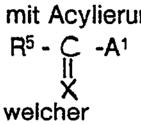 (V)

in welcher
$R^5$ und X die oben angegebene Bedeutung haben und
$A^1$ für Halogen oder den Rest $R^5$-CO-O-steht, wobei
$R^5$ die oben angegebene Bedeutung hat, oder
β) mit Alkylierungsmitteln der Formel (VI),

$R^{4-2}$ -$A^2$ (VI)

in welcher
$R^{4-2}$ die oben angegebene Bedeutung hat und
$A^2$ für eine elektronenanziehende Abgangsgruppe steht,
jeweils gegebenenfalls in Gegenwart eines Verdünnungsmittels und gegebenenfalls in Gegenwart eines Säurebindemittels sowie gegebenenfalls in Gegenwart eines Katalysators umsetzt;

d) man erhält 5-Amino-1-aryl-3-tert.-butyl-pyrazole der Formel (Id),

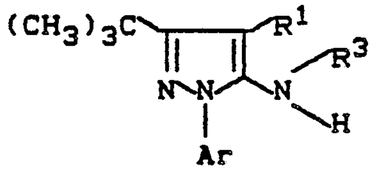

(Id)

in welcher
$R^1$, $R^3$ und Ar die oben angegebene Bedeutung haben,
auch, wenn man die nach den Verfahren (a) oder (c) erhältlichen 1-Aryl-3-tert.-butyl-pyrazole der Formel (Ie),

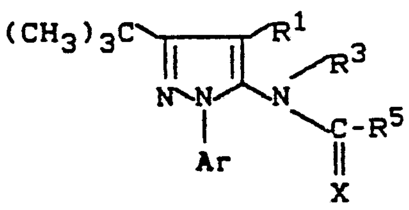

(Ie)

in welcher
$R^1$, $R^3$, $R^5$, X und Ar die oben angegebene Bedeutung haben,
gegebenenfalls in Gegenwart eines Verdünnungsmittels und gegebenenfalls in Gegenwart eines Katalysators deacyliert;
e) man erhält 1-Aryl-3-tert.-butyl-pyrazole der Formel (If),

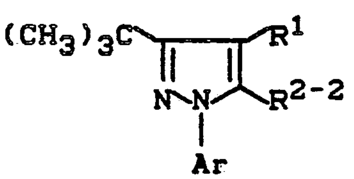

(If)

in welcher
$R^1$ und Ar die oben angegebene Bedeutung haben und
$R^{2-2}$ für Halogen steht, oder auch für Wasserstoff steht, wenn $R^1$ Halogen bedeutet,
wenn man die nach den Verfahren (a), (b) oder (d) erhältlichen 5-Amino-1-aryl-3-tert.-butyl-pyrazole der Formel (Ig),

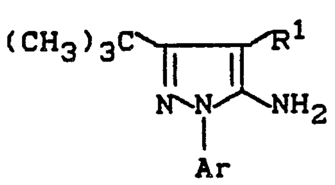

(Ig)

in welcher
$R^1$ und Ar die oben angegebene Bedeutung haben,
mit einem anorganischen oder organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

Schließlich wurde gefunden, daß die neuen 1-Aryl-3-tert.-butyl-pyrazole der allgemeinen Formel (I) starke insektizide und akarizide Eigenschaften besitzen.

Überraschenderweise zeigen die erfindungsgemäßen 1-Aryl-3-tert.-butyl-pyrazole der allgemeinen Formel (I) eine erheblich bessere insektizide und akarizide Wirksamkeit, als die aus dem Stand der Technik bekannten Pyrazol-Derivate, wie beispielsweise das 4-Cyano-5-propionamido-1-(2,4,6-trichlorphenyl)-pyrazol, das 5-Dimethylaminocarbonyloxy-1-isopropyl-3-methylsulfinylmethyl-pyrazol oder das 1-Cyclohexyl-5-dimethylaminocarbonyloxy-3-methylthiomethyl-pyrazol, welches chemisch bzw. wirkungsmäßig naheliegende Verbindungen sind.

Die neuen substituierten 1-Aryl-3-tert.-butyl-pyrazole sind durch die Formel (I) allgemein definiert. Hierbei stehen vorzugsweise

$R^1$ für Nitro, Fluor, Chlor, Brom oder Jod;

$R^2$ für Fluor, Chlor, Brom, Jod oder die Gruppierung $-NR^3R^4$; oder auch für Wasserstoff, wenn $R^1$ für ein Halogen steht, wobei

$R^3$ für Wasserstoff oder für jeweils geradkettiges oder verzweigtes, gegebenenfalls einfach oder mehrfach, gleich oder verschieden substituiertes Alkyl, Alkenyl und Alkinyl mit jeweils bis zu 8 Kohlenstoffatomen steht, wobei als Substituenten jeweils infrage kommen: Halogen, Cyano, Nitro, Hydroxy, Mercapto, Carboxy, jeweils geradkettiges oder verzweigtes Alkoxy, Alkylthio und Alkoxycarbonyl mit jeweils bis zu 6 Kohlenstoffatomen; ferner für jeweils gegebenenfalls einfach oder mehrfach, gleich oder verschieden substituiertes Cycloalkyl mit 3 bis 7 Kohlenstoffatomen und Cycloalkylalkyl mit 3 bis 7 Kohlenstoffatomen im Cycloalkylteil und 1 bis 4 Kohlenstoffatomen im Alkylteil steht, wobei als Substituenten im Cycloalkylteil jeweils in Frage kommen: Halogen und geradkettiges oder verzweigtes Alkyl mit 1 bis 4 Kohlenstoffatomen; und

$R^4$ unabhängig von $R^3$ für die gleichen Reste wie $R^3$ steht, oder zusätzlich für einen Rest $-C(X)-R^5$ steht, wobei

X für Säuerstoff oder Schwefel steht und

$R^5$ für Wasserstoff, für geradkettiges oder verzweigtes Alkyl mit 1 bis 12 Kohlenstoff atomen, für jeweils geradkettiges oder verzweigtes Alkenyl oder Alkinyl, mit jeweils 2 bis 4 Kohlenstoffatomen, für jeweils geradkettiges oder verzweigtes Alkoxyalkyl, Alkylthioalkyl, Alkoxy, Alkylthio, Alkylamino, Dialkylamino oder Halogenalkyl mit jeweils 1 bis 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, oder für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen; sowie

Ar für jeweils gegebenenfalls einfach oder mehrfach gleich oder verschieden substituiertes Phenyl (ausgenommen Dinitrophenyl), 2-Pyridyl, 3-Pyridyl oder 4-Pyridyl steht, wobei als Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^6$, wobei

$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für eine Zahl 0, 1 oder 2 steht.

Besonders bevorzugt sind substituierte 1-Aryl-3-tert.-butyl-pyrazole der Formel (I), bei welchen

$R^1$ für Nitro, Chlor oder Brom steht;

$R^2$ für Chlor, Brom oder die Gruppierung $-NR^3R^4$ steht; oder auch für Wasserstoff steht, wenn $R^1$ für ein Halogen steht, wobei

$R^3$ für jeweils gegebenenfalls ein-bis dreifach, gleich oder verschieden substituiertes Methyl, Ethyl, n-oder i-Propyl, n-, i-, s-oder t-Butyl, Allyl, Butenyl, Propargyl oder Butinyl steht, wobei als Substituenten infrage kommen: Fluor, Chlor, Brom, Iod, Cyano, Nitro, Hydroxy, Mercapto, Carboxy, Methoxy, Ethoxy, n-oder i-Propoxy, Methylthio, Ethylthio, n-oder i-Propylthio, Methoxycarbonyl, Ethoxycarbonyl, n-oder i-Propoxycarbonyl, n-, i-, s-oder t-Butoxycarbonyl; ferner für jeweils gegebenenfalls einfach bis dreifach, gleich oder verschieden im Cycloalkylteil durch Chlor, Methyl oder Ethyl substituiertes Cyclopropyl, Cyclopropylmethyl, Cyclohexylmethyl, Cyclohexylethyl, Cycloheptyl oder Cycloheptylmethyl;

$R^4$ unabhängig von $R^3$ für die gleichen Reste wie $R^3$ steht, oder zusätzlich für einen Rest $-\underset{\underset{X}{\|}}{C}-R^5$ steht, wobei

X für Sauerstoff oder Schwefel steht und

$R^5$ für Wasserstoff, Methyl, Ethyl, n-oder i-Propyl, n-, i-, s-oder t-Butyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht; sowie

Ar für gegebenenfalls ein-bis fünffach, gleich oder verschieden substituiertes Phenyl (ausgenommen Dinitrophenyl) oder für jeweils gegebenenfalls ein-bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei

$R^6$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und

m für eine Zahl 0, 1 oder 2 steht.

Im einzelnen seien außer den bei den Herstellungsbeispielen genannten Verbindungen die folgenden substituierten 1-Aryl-3-tert.-butyl-pyrazole der allgemeinen Formel (I) genannt:

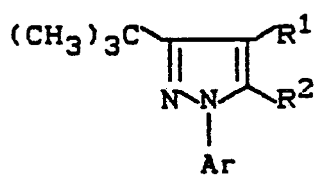

(If)

| $R^1$ | $R^2$ | Ar |
|---|---|---|
| Br | H | 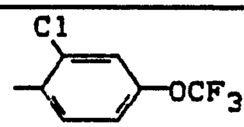 |
| Cl | H | 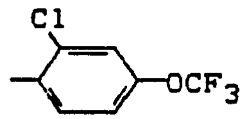 |
| $NO_2$ | $-NH_2$ | 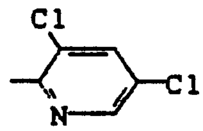 |
| $NO_2$ | $-NHCH_3$ | 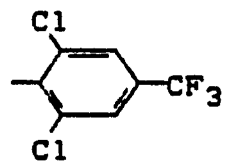 |
| $NO_2$ | $-NHCH_2CH_2OCH_3$ | 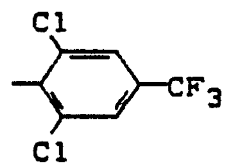 |
| Br | $-N(CH_3)_2$ | 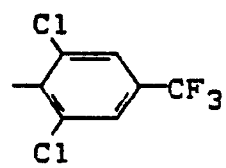 |
| $NO_2$ | 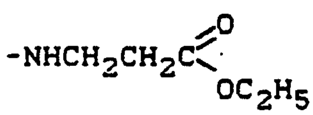 | 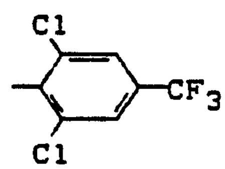 |
| $NO_2$ | Br | 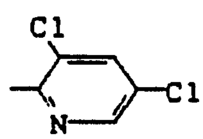 |

| $R^1$ | $R^2$ | Ar |
|---|---|---|
| $NO_2$ | Cl | 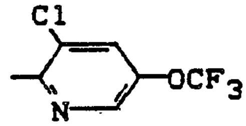 |
| Cl | Cl | 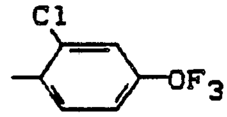 |
| Cl | 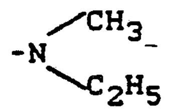 | 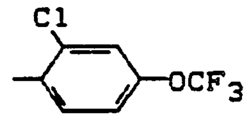 |
| $NO_2$ | $-NHCH_2SCH_3$ | 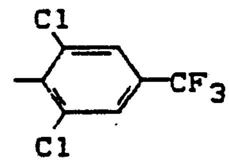 |

Verwendet man beispielsweise 1-(2,6-Dichlor-4-trifluormethyl-phenyl)-5-propionamido-3-tert.-butyl-pyrazol und Salpetersäure als Ausgangsstoffe, so läßt sich der Reaktionsablauf des erfindungsgemäßen Verfahrens (a) durch das folgende Formelschema darstellen:

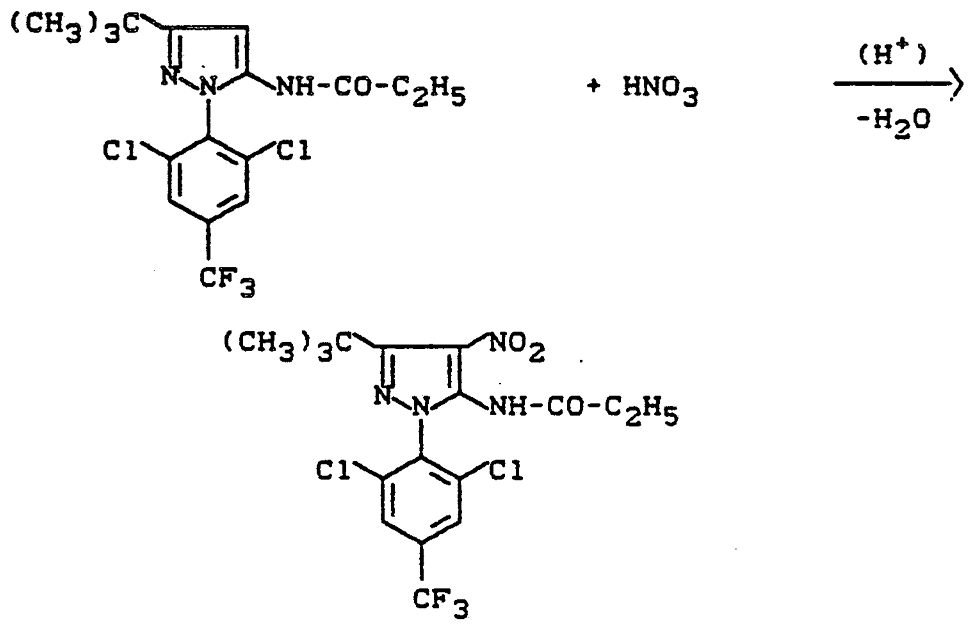


Verwendet man beispielsweise 5-Brom-1-(2,6-dichlor-4-trifluormethyl-phenyl)-4-nitro-3-tert.-butyl-pyrazol und Isopropylamin als Ausgangsstoffe, so läßt sich der Reaktionsablauf des erfindungsgemäßen Verfahrens (b) durch das folgende Formelschema darstellen:

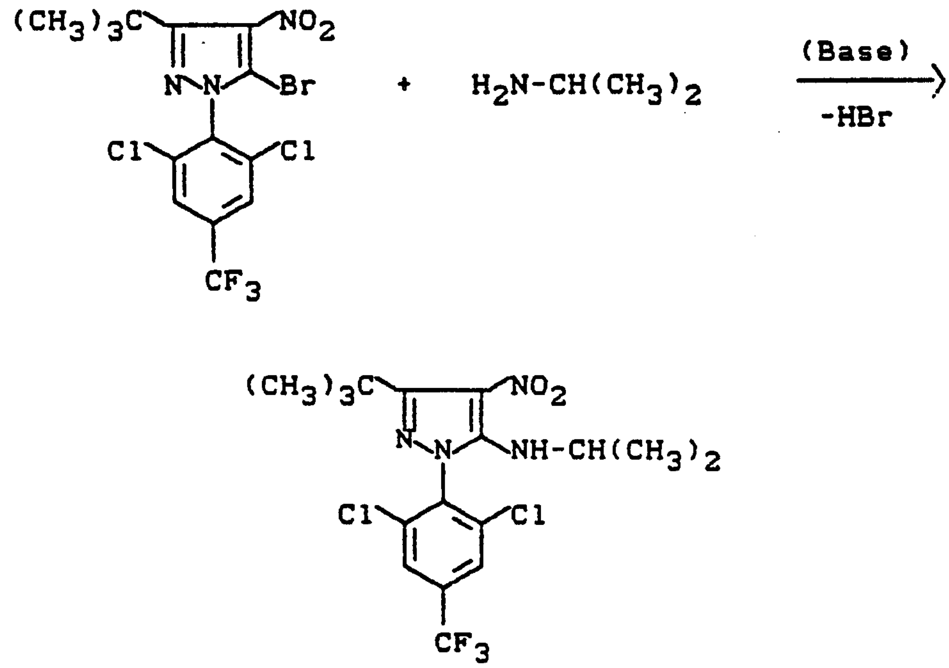


Verwendet man beispielsweise 5-Amino-1-(2,6-dichlor-4-trifluormethyl-phenyl)-4-nitro-3-tert.-butyl-pyrazol und Butansäurechlorid als Ausgangsstoffe, so läßt sich der Reaktionsablauf des erfindungsgemäßen Verfahrens (c/α) durch das folgende Formelschema darstellen:

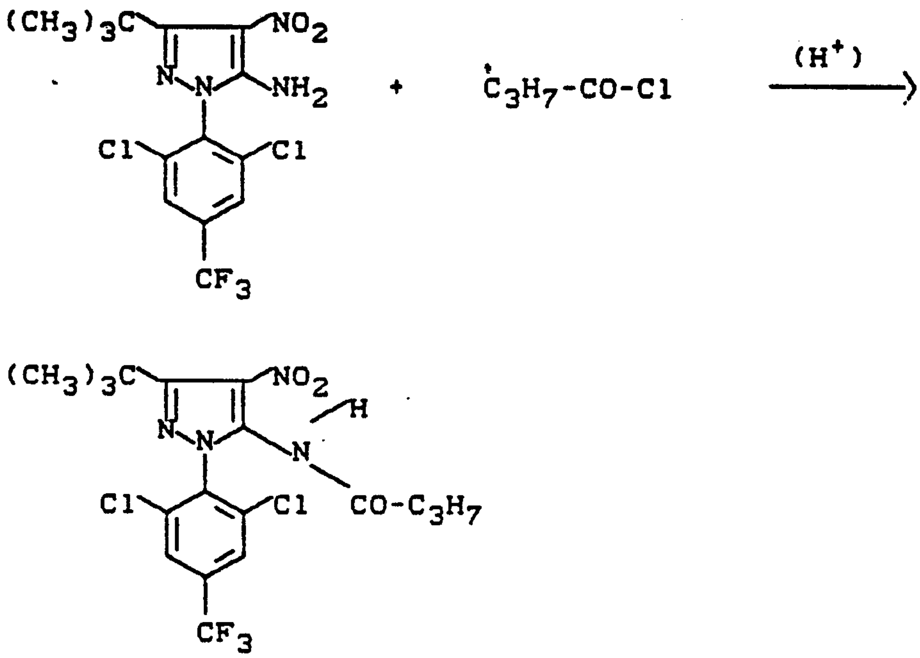


Verwendet man beispielsweise 5-Amino-1-(2,6-dichlor-4-trifluormethyl-phenyl)-4-nitro-3-tert.-butyl-pyrazol und Dimethylsulfat als Ausgangstoffe, so läßt sich der Reak tionsablauf des erfindungsgemäßen Verfahrens (c/β) durch das folgende Formelschema darstellen:

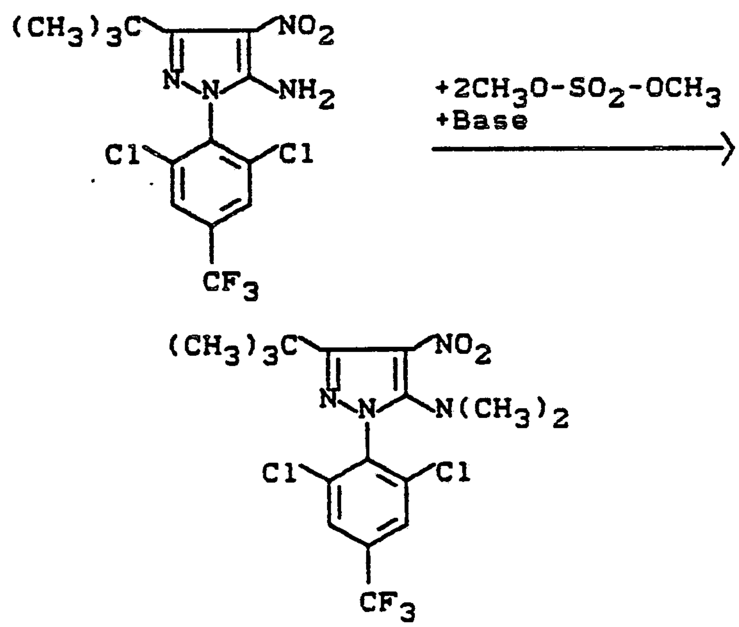


Verwendet man beispielsweise 5-Acetylamino-4-chlor-1-(2,6-dichlor-4-trifluormethyl-phenyl)-3-tert.-butyl-pyrazol als Ausgangsstoff, so läßt sich der Reaktionsablauf des erfindungsgemäßen Verfahrens (d) durch das folgende Formelschema darstellen:

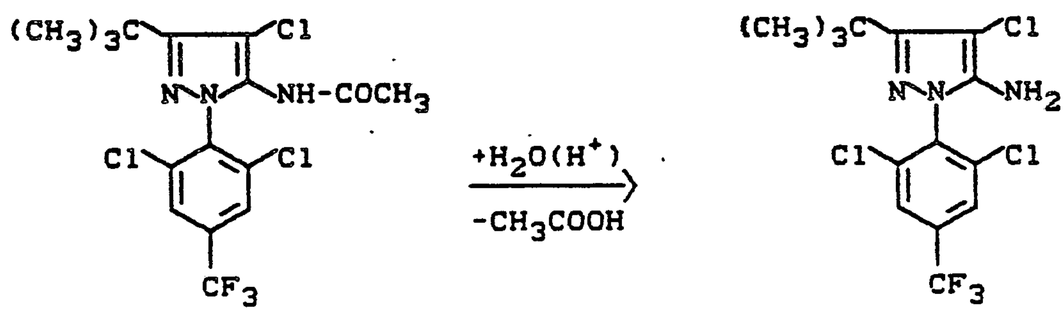


Verwendet man beispielsweise 5-Amino-1-(2,6-dichlor-4-trifluormethyl-phenyl)-4-nitro-3-tert.-butyl-pyrazol und tert.-Butyl-nitrit/Bromoform als Ausgangsstoffe, so läßt sich der Reaktionsablauf des erfindungsgemäßen Verfahrens (e) durch das folgende Formelschema darstellen:

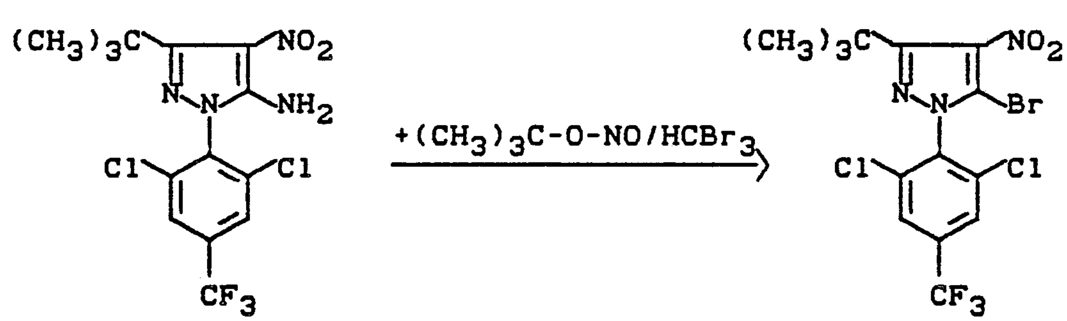

Die zur Durchführung des erfindungsgemäßen Verfahrens (a) als Ausgangsstoffe benötigten 1-Aryl-3-tert.-butyl-pyrazole sind durch die Formel (II) allgemein definiert. In dieser Formel steht Ar vorzugsweise für diejenigen Reste, die bereits im Zusammenhang mit der Beschreibung der erfindungsgemäßen Stoffe der Formel (I) als bevorzugt für diesen Substituenten genannt wurden. $R^{2-1}$ steht vorzugsweise für Wasserstoff, Fluor, Chlor, Brom, Jod sowie die Gruppierung $-NR^3R^4$, wobei $R^3$ und $R^4$ vorzugsweise für diejenigen Reste stehen, die bereits im Zusammenhang mit der Beschreibung der erfindungsgemäßen Stoffe der Formel (I) als bevorzugt für diese Substituenten genannt wurden.

Die 1-Aryl-3-tert.-butyl-pyrazole der Formel (II) sind noch nicht bekannt; sie können jedoch in allgemein bekannter Art und Weise erhalten werden. Die Vorprodukte der Formel (II) zeigen ebenfalls eine insektizide Wirksamkeit.

Man erhält 1-Aryl-3-tert.-butyl-pyrazole der Formel (IIa),

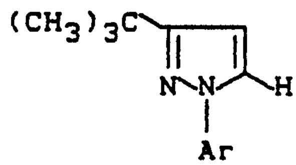

(IIa)

in welcher
Ar die oben angegebene Bedeutung hat,
beispielsweise, wenn man (2,2,-Dimethoxy)-ethyl-tert.-butyl-keton der Formel (VII),

$(CH_3)_3C-CO-CH_2CH(OCH_3)_2$ (VII)

mit Hydrazinen der Formel (VIII),

$Ar-NH-NH_2$ (VIII)

in welcher
Ar die oben angegebene Bedeutung hat,
gegebenenfalls in Gegenwart eines Verdünnungsmittels, wie beispielsweise Ethylenglykolmonoethylether oder Ethanol, bei Temperaturen zwischen -50 °C und + 150 °C cyclisiert (vgl. hierzu auch die deutsche Patentanmeldung P 35 09 567 vom 16.03.1985 [LeA 23 595]).

Das (2,2-Dimethoxy)-ethyl-tert.butyl-keton der Formel (VIII) ist bekannt (vgl. Chemistry Letters 1978, S. 263).

Die Hydrazine der Formel (VIII) sind größtenteils bekannt oder können nach bekannten Verfahren in analoger Art und Weise hergestellt werden (vgl. z.B. Houben-Weyl, Methoden der organischen Chemie; Band X/2, S. 203, Thieme Verlag Stuttgart 1967).

Man erhält 1-Aryl-5-halogen-3-tert.-butyl-pyrazole der Formel (IIb),

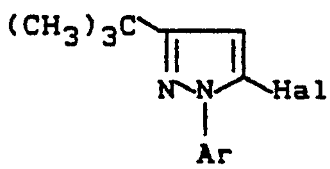

(IIb)

in welcher
Ar und Hal die oben angegebene Bedeutung haben, beispielsweise, wenn man Pyrazolin-5-one der Formel (IX),

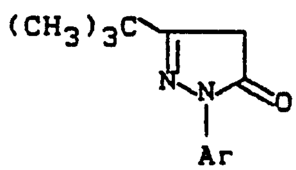

(IX)

in welcher
Ar die oben angegebene Bedeutung hat,
mit Phosphoroxyhalogeniden bei Temperaturen zwischen 100 und 250 °C umsetzt (vgl. auch die deutsche

Patentanmeldung P 35 20 329 vom 07.06.1985 [LeA 23 639]).

Die Pyrazolin-5-one der Formel (IX) werden erhalten, indem man tert.-Butyl-carbonyl-essigsäuremethylester mit Hydrazinen der Formel (VIII) gegebenenfalls in Gegenwart eines Verdünnungsmittels, wie beispielsweise Toluol oder Ethanol und gegebenenfalls in Gegenwart eines Katalysators, wie beispielsweise p-Toluolsulfonsäure,bei Temperaturen zwischen +50 °C und +150 °C cyclisiert.

Man erhält 5-Amino-1-aryl-3-tert.-butyl-pyrazole der Formel (IIc),

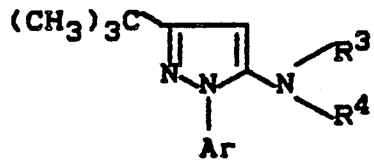
(IIc)

in welcher
Ar, $R^3$ und $R^4$ die oben angegebene Bedeutung haben,
beispielsweise, wenn man 5-Amino-1-aryl-3-tert.-butyl-pyrazole der Formel (IIe),

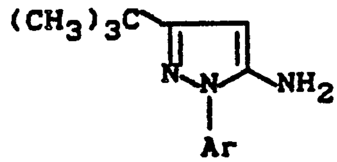
(IIe)

in welcher
Ar die oben angegebene Bedeutung hat,
mit Acylierungsmitteln der Formel (V) bzw. mit Alkylierungsmitteln der Formel (VI) entsprechend dem erfindungsgemäßen Verfahren (c) umsetzt.

Die 5-Amino-1-aryl-3-tert.-butyl-pyrazole der Formel (IIe) können erhalten werden, wenn man Cyanpinakolin [$(CH_3)_3C$-CO-$CH_2CN$; vgl. Ber. 44, 2065 (1911)] mit Hydrazinen der Formel (VIII), gegebenenfalls in Gegenwart eines Verdünnungsmittels, wie beispielsweise Ethylenglykolmonoethylether oder Ethanol, bei Temperaturen zwischen +50 °C und +150 °C cyclisiert.

Die außerdem zur Durchführung des erfindungsgemäßen Verfahrens (a) als Ausgangsstoffe benötigten Halogenierungs-oder Nitrierungsmittel sind durch die Formel (III) allgemein definiert. In dieser Formel steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden. A steht vorzugsweise für Halogen, insbesondere für Chlor oder Brom. Weiterhin verwendbare elektrophile Reagenzien sind Sulfurylchlorid, Phosphoroxychlorid, Nitriersäure und andere üblicherweise zu verwendende Nitrierungsmittel.

Die Halogenierungs-und Nitrierungsmittel der Formel (III) sind allgemein bekannte Verbindungen der organischen Chemie.

Die zur Durchführung des erfindungsgemäßen Verfahrens (b) als Ausgangsstoffe benötigten 1-Aryl-5-halogen-3-tert.-butyl-pyrazole sind durch die Formel (Ib) allgemein definiert. In dieser Formel stehen Ar und $R^1$ vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Brom oder Chlor.

Die 1-Aryl-5-halogen-3-tert.-butyl-pyrazole der Formel (Ib) sind erfindungsgemäße Verbindungen und erhältlich mit Hilfe des erfindungsgemäßen Verfahrens (a).

Die außerdem zur Durchführung des erfindungsgemäßen Verfahrens (b) als Ausgangstoffe benötigten Amine sind durch die Formel (IV) allgemein definiert. In dieser Formel steht $R^3$ vorzugswiese für diejenigen Reste, die bereits im Zusammenhang mit der Beschreibung der erfindungsgemäßen Stoffe der Formel (I) als bevorzugt für diesen Substituenten genannt wurden. $R^{4-1}$ steht unabhängig von $R^3$ vorzugsweise für die gleichen Reste wie $R^3$.

Die Amine der Formel (IV) sind allgemein bekannte Verbindungen der organischen Chemie.

Die zur Durchführung des erfindungsgemäßen Verfahrens (c) als Ausgangsstoffe benötigten 5-Amino-1-aryl-3-tert.-butyl-pyrazole sind durch die Formel (Id) allgemein definiert. In dieser Formel stehen Ar, $R^1$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-3-tert.-butyl-pyrazole der Formel (Id) sind erfindungsgemäße Verbindungen und erhältlich mit Hilfe der erfindungsgemäßen Verfahren (a) und (b).

Die außerdem zur Durchführung des erfindungsgemäßen Verfahrens (c)/Variante $\alpha$ als Ausgangsstoffe benötigten Acylierungsmittel sind durch die Formel (V) allgemein definiert. In dieser Formel stehen $R^5$ und X vorzugsweise für diejenigen Reste, die bereits im Zusammenhang mit der Beschreibung der erfindungsgemäßen Stoffe der Formel (I) als bevorzugt für diese Substituenten genannt wurden. $A^1$ steht vorzugsweise für Chlor oder Brom oder für einen Rest $R^5$-CO-O-.

Die außerdem zur Durchführung des erfindungsgemäßen Verfahrens (c)/Variante $\beta$ als Ausgangsstoffe benötigten Alkylierungsmittel sind durch die Formel (VI) allgemein definiert. In dieser Formel steht $R^{4-2}$ vorzugsweise für diejenigen Reste, die bereits im Zusammenhang mit der Beschreibung der erfinungsgemäßen Stoffe der Formel (I) vorzugsweise für $R^4$, ausgenommen Wasserstoff, genannt wurden. $A^2$ steht vorzugsweise für Chlor, Brom oder Jod sowie für jeweils gegebenenfalls substituiertes Alkylsulfonyloxy, Alkoxysulfonyloxy oder Arylsulfonyloxy, wie beispielsweise Methoxysulfonyloxy oder p-Tolylsulfonyloxy.

Die Acylierungsmittel der Formel (V) und die Alkylierungsmittel der Formel (VI) sind allgemein bekannte Verbindungen der organischen Chemie.

Die zur Durchführung des erfindungsgemäßen Verfahrens (d) als Ausgangstoffe benötigten 1-Aryl-3-tert.-butyl-pyrazole sind durch die Formel (Ie) allgemein definiert. In dieser Formel stehen $R^1$, $R^3$, $R^5$, X und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Aryl-3-tert.-butyl-pyrazole der Formel (Ie) sind erfindungsgemäße Verbindungen und erhältlich mit Hilfe der erfindungsgemäßen Verfahren (a) und (c).

Die zur Durchführung des erfindungsgemäßen Verfahrens (e) als Ausgangsstoffe benötigten 5-Amino-1-aryl-3-tert.-butyl-pyrazole sind durch die Formel (Ig) allgemein definiert. In dieser Formel stehen $R^1$ und Ar vozugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-3-tert.-butyl-pyrazole der Formel (Ig) sind erfindungsgemäße Verbindungen und erhältlich mit Hilfe der erfindungsgemäßen Verfahren (a), (b) und (d).

Als Verdünnungsmittel zur Durchführung des erfindungsgemäßen Verfahrens (a) kommen alle üblicherweise für derartige elektrophile Substitutionen verwendbaren Lösungsmittel in Frage, Vorzugsweise verwendet man die als Reagenzien in Frage kommenden Säuren oder Gemische, wie beispielsweise Schwefelsäure, Salpetersäure, Sulfurylchlorid, Phosphoroxychlorid/Dimethylformamid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des Herstellungsverfahrens (a) kommen ebenfalls die für derartige Reakionen üblichen Katalysatoren in Frage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (a) in einem größeren Bereich variiert werden. Im allgemeinen arbeitet man zwischen -50°C und ±200°C, vorzugsweise zwischen -20 und +150°C.

Zur Durchführung des Herstellungsverfahrens (a) setzt man pro Mol 1-Aryl-3-tert.-butyl-pyrazol der Formel (II), im allgemeinen 1,0 bis 10,0 Mol, vorzugsweise 1,0 bis 5,0 Mol an elektrophilem Agens der Formel (III) und gegebenenfalls 0,1 bis 10 Mol an Katalysator oder Reaktionshilfsmittel ein. Die Reaktionsführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) kann gegebenenfalls in Gegenwart eines geeigneten Säurebindemittels durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (IV) gleichzeitig als Säurebindemittel zu verwenden.

Die Reaktionstemperaturen können bei der Durchführung des erfindungsgemäßen Verfahrens (b) in einem größeren Bereich variiert werden. Im allgemeinen arbeitet man bei Temperaturen zwischen -20° C und +200° C, vorzugsweise bei Temperaturen zwischen 0° C und +150° C.

Zur Durchführung des erfindungsgemäßen Verfahrens (b) setzt man pro Mol an 1-Aryl-5-halogen-3-tert.-butyl-pyrazol der Formel (Ib) im allgemeinen 1,0 bis 10,0 Mol, vorzugsweise 1,0 bis 5,0 Mol an Amin der Formel (IV) ein. Die Reaktionsdurchführung, Aufarbeitung und Isolierung der Reaktionsprodukte der Formel (Ia) erfolgt nach allgemein üblichen Verfahren.

Als Verdünnungsmittel zur Durchführung des erfindungsgemäßen Verfahrens (c) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-ammoniummethylsulfat, Dimeth yl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (c) kommen alle üblicherweise verwendbaren anorganischen und organischen Basen infrage. Vorzugsweise verwendet man Alkalimetallhydride, -hydroxide, - amide, -alkoholate, -carbonate oder -hydrogencarbonate, wie beispielsweise Natriumhydrid, Natriumamid, Natriummethylat, Natriumhydroxid, Natriumcarbonat oder Natriumhydrogencarbonat oder auch tertiäre Amine, wie bespielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (c) in einem größeren Bereich variiert werden. Im allgemeinen arbeitet man zwischen -20 °C und + 150 °C, vorzugsweise zwischen 0 °C und +100 °C.

Zur Durchführung des Herstellungsverfahrens (c) setzt man pro Mol 5-Amino-1-aryl-3-tert.-butyl-pyrazol der Formel (Id) im allgemeinen 1.0 bis 20.0 Mol, vorzugsweise 1,0 bis 15.0 Mol an Acylierungsmittel der Formel (V) oder an Alkylierungsmittel der Formel (VI) und gegebenenfalls 1.0 bis 3.0 Mol, vorzugsweise 1,0 bis 2,0 Mol an Säurebindemittel sowie gegebenenfalls 0.01 bis 1.0 Mol an Phasentransferkatalysator ein. Die Reaktionsdurchführung, Aufarbeitung und Isolierung der Reaktionsprodukte der Formel (Ic) erfolgt in allgemein üblicher Art und Weise.

Als Verdünnungsmittel zur Durchführung des erfindungsgemäßen Verfahrens (d) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Katalysatoren zur Durchführung des Herstellungsverfahrens (d) kommen vorzugsweise Säuren, insbesondere Chlorwasserstoffsäure oder Schwefelsäure sowie Basen, insbesondere Natriumhydroxid, Natriumhydrid und Kalium-tert.-butyl in Frage.

Die Reaktionstemperaturen können bei der Durchführung des Herstellungsverfahrens (d) in einem größeren Bereich variiert werden. Im allgemeinen arbeitet man zwischen +20° C und +150° C, vorzugsweise zwischen +50° C und +120° C.

Zur Durchführung des Herstellungsverfahrens (d) setzt man pro Mol 1-Aryl-3-tert.-butyl-pyrazol der Formel (Ie) im allgemeinen 1,0 bis 20,0 Mol, vorzugsweise 1,0 bis 10,0 Mol an Katalysator ein und erwärmt für mehrere Stunden auf die erforderliche Reaktionstemperatur. Die Aufarbeitung, Isolierung und Reinigung der Reaktionsprodukte der Formel (Id) erfolgt nach üblichen Methoden.

Das erfindungsgemäße Verfahren (e) wird in Gegenwart eines anorganischen oder organischen Nitrits durchgeführt. Als solche kommen alle üblicherweise für derartige Diazotierungsreaktionen üblichen Nitritverbindungen in Frage. Besonders bevorzugt verwendet man Alkalimetallnitrite, wie beispielsweise Natriumnitrit oder Alkylnitrite wie beispielsweise t-Buylnitrit oder Isopentylnitrit.

Das erfindungsgemäße Verfahren (e) wird gegebenenfalls in Gegenwart einer Halogenwasserstoffsäure durchgeführt. In diesem Fall erhält man als Reaktionsprodukte 1-Aryl-pyrazole der Formel (If), bei welchen der Rest $R^{2-2}$ für einen Halogenrest steht, der dem Anion der verwendeten Halogenwasserstoffsäure entspricht. Vorzugsweise verwendet man jeweils wässrige oder auch nicht wässrige Lösungen der Fluorwasserstoffsäure, Chlorwasserstoffsäure, Bromwasserstoffsäure oder Iodwasserstoffsäure.

Als Verdünnungsmittel zur Durchführung des erfindungsgemäßen Verfahrens (e) kommen alle üblicherweise für derartige Diazotierungsreaktionen üblichen Lösungsmittel in Frage. Vorzugsweise verwendet man Halogenkohlenwasserstoffe wie Chloroform oder Bromoform oder wässrige Säuren wie beispielsweise Halogenwasserstoffsäuren oder Schwefelsäure, wobei die Säurekomponente gleichzeitig als Reagenz und/oder als Reaktionshilfsmittel fungiert. Bei der Verwendung von Bromoform als Verdünnungsmittel erhält man in der Regel die entsprechenden 5-Brom-pyrazole, wobei das Bromoform gleichzeitig als Verdünnungsmittel und als Reagenz fungiert. Die entsprechende Reaktion in Gegenwart von Chloroform als Verdünnungsmittel ergibt im allgemeinen eine Mischung von 5-Chlor-pyrazolverbindungen der Formel (If) und den analogen reduzierten Verbindungen der Formel (If), die in der 5-Position des Pyrazolringes einen Wasserstoffrest tragen. Diese Mischungen lassen sich mit üblichen Trennmethoden, wie beispielsweise destillativ oder chromatographisch, auftrennen.

Das erfindungsgemäße Verfahren (e) wird üblicherweise in Gegenwart eines Reaktionshilfsmittels durchgeführt. Als solche kommen insbesondere starke Mineralsäuren wie Schwefelsäure oder Phosphorsäure oder die oben aufgeführten Halogenwasserstoffsäuren in Frage, die in diesem Fall gleichzeitig als Reagenz und als Katalysator wirken.

Das erfindungsgemäße Verfahren (e) kann gegebenenfalls in Gegenwart eines geeigneten Reduktionsmittels durchgeführt werden. In diesem Fall erhält man als Reaktionsprodukte 1-Aryl-pyrazole der Formel (If), bei welchen der Rest $R^{2-2}$ für Wasserstoff steht. Als Reduktionsmittel verwendet man in diesen Fällen besonders bevorzugt unterphosphorige Säure ($H_3PO_2$).

Die Reaktionstemperaturen können bei der Durchführung des erfindungsgemäßen Verfahrens (e) in einem größeren Bereich variiert werden. Im allgemeinen arbeitet man bei Temperaturen zwischen -30° C und +60° C, vorzugsweise bei Temperaturen zwischen -20° C und +40° C.

Zur Durchführung des erfindungsgemäßen Verfahrens (e) setzt man pro Mol an 5-Amino-1-aryl-3-tert.-butyl-pyrazol der Formel (Ig) im allgemeinen 1,0 bis 1,8 Mol an Nitrit, gegebenenfalls 1,0 bis 20,0 Mol, vorzugsweise 1,0 bis 10,0 Mol an Halogenwasserstoffsäure, gegebenenfalls 1,0 bis 50,0 Mol, vorzugsweise 1,0 bis 20,0 Mol an Reduktionsmittel und gegebenenfalls 1,0 bis 20,0 Mol, vorzugsweise 1,0 bis 10,0 Mol an als Reaktionshilfsmittel verwendete Mineralsäure ein.

Dabei setzt man üblicherweise der Reaktionsmischung be stehend aus 5-Amino-1-aryl-3-tert.-butylpyrazol der Formel (Ig), Mineralsäure, Verdünnungsmittel und Halogenwasserstoffsäure bzw. Reduktionsmittel das Nitrit in kleinen Portionen gegebenenfalls in geeignetem Verdünnungsmittel gelöst zu.

Die Aufarbeitung und Isolierung der Reaktionsprodukte der Formel (If) erfolgt nach üblichen Methoden z.B. durch Abfiltrieren von kristallinen Produkten oder durch Extraktion mit einem geeigneten organischen Lösungsmittel. Die Identifizierung erfolgt durch Schmelzpunkt oder Protonen-Kernresonanz-Spektrum.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats-und Materialschutz sowie auf dem Hygienesketor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae,

Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Litho colletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocaptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene-und Vorratsschädlinge, sondern auch auf dem veterinärmedizinischen Sektor gegen tierische Parasiten (Ektoparasiten) wie Schildzecken, Lederzecken, Räubemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen Wirkstoffe zeichnen sich durch eine hohe insektizide und akarizide Wirksamkeit aus. Sie lassen sich mit besonders gutem Erfolg gegen pflanzenschädigende Insekten, wie beispielsweise gegen die Larven der Meerrettichblattkäfer (Phaedon cochleariae)sowie gegen pflanzenschädigende Milben, wie beispielsweise gegen die gemeine Spinnmilbe (Tetranychus urticae) einsetzen.

Außerdem besitzen die erfindungsgemäßen Wirkstoffe eine hohe Wirkung gegen Hygiene-und Vorratsschädlinge und lassen sich beispielsweise zur Bekämpfung der Stubenfliege (Musca domestica), zur Bekämpfung des gemeinen Kornkäfers (Sitophilus granarius) oder zur Bekämpfung der Hausschabe (Blatella germanica) einsetzen. Darüber hinaus lassen sich die erfindungsgemäßen Wirkstoffe mit besonders

gutem Erfolg zur Bekämpfung von parasitisch lebenden Warmblüterschädlingen wie beispielsweise gegen Weideviehfliegen (Musca autumnalis) oder gegen Stechfliegen (Stomoxys calcitrans) einsetzen. Daneben besitzen die erfindungsgemäßen Wirkstoffe in entsprechenden Aufwandmengen auch eine gute fungizide Wirksamkeit und lassen sich beispielsweise zur Bekämpfung von Oomyceten-Arten einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brenn sätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Herstellungsbeispiele

Beispiel 1

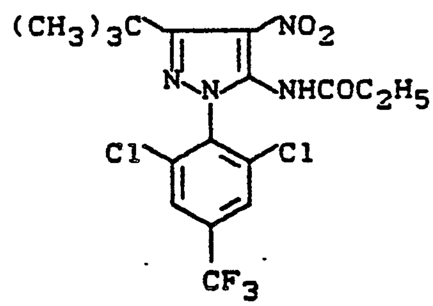

(I-1)

**(Verfahren a)**

Zu einer Suspension aus 61,2 g (0,15 Mol) 3-tert.-Butyl-5-propionamido-1-(2,6-dichlor-4-trifluormethylphenyl)-pyrazol in 500 ml Acetanhydrid werden bei Raumtemperatur 14,2 g (0,225 Mol) 98-%ige Salpetersäure getropft, wobei die Temperatur auf 40 °C ansteigt. Man läßt über Nacht bei Raumtemperatur nachrühren und gibt anschließend die Reaktionsmischung auf Eiswasser. Das auskristallisierte Produkt wird abgesaugt mit Petrolether verrieben und getrocknet.

Man erhält 56,2 g (94,4 % der Theorie) 3-tert.-Butyl-4-nitro-5-propionamido-1-(2,6-dichlor-4-trifluormethylphenyl)-pyrazol vom Schmelzpunkt 151 - 152 °C.

Herstellung der Ausgangsverbindung

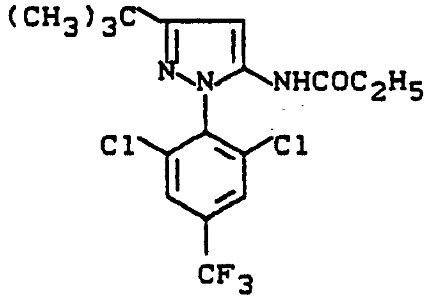

(II-1)

Zu einer Lösung aus 119,5 g (0,34 Mol) 3-tert.-Butyl-5-amino-1-(2,6-dichlor-4-trifluormethylphenyl)-pyrazol und 30 g (0,38 Mol) Pyridin in 500 ml trockenem Methylenchlorid werden 31,4 g (0,34 Mol) Propionylchlorid getropft. Man läßt bei Raumtemperatur nachrühren und wäscht dann die Reaktionslösung 3 mal mit 2 N HCl, 1 mal mit Wasser, 2 mal mit gesättigter Natriumhydrogencarbonatlösung, 1 mal mit Wasser, trocknet über Magnesiumsulfat und engt ein.

Nach Umkristallisation aus Cyclohexan/Toluol erhält man 120,7 g (87 % der Theorie) 3-tert.-Butyl-5-propionamido-1-(2,6-dichlor-4-trifluormethylphenyl)-pyrazol vom Schmelzpunkt 193 °C.

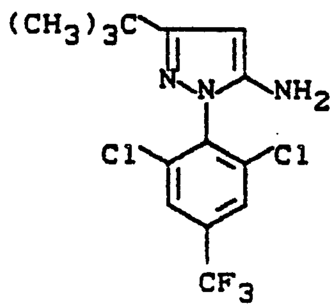

(II-2)

122,5 g (0,5 Mol) 2,6-Dichlor-4-trifluormethyl-phenylhydrazin und 68,8 g (0,55 Mol) 1-Cyan-3,3-dimethyl-2-butanon werden in 1 l Ethanol gelöst und 24 Stunden bei Siedetemperatur erhitzt. Anschließend werden 5 ml konzentrierte Schwefelsäure zugesetzt und weitere 23 Stunden unter Rückfluß gerührt. Nach beendeter Reaktion wird das Lösungsmittel abdestilliert, der Rückstand in Chloroform aufgenommen und mit 25-%iger wässriger Ammoniaklösung alkalisch gestellt. Die organische Phase wird abgetrennt und die wäßrige mit Chloroform extrahiert. Die vereinigten organischen Phasen werden über Magnesiumsulfat getrocknet und im Vakuum vom Lösungsmittel befreit.

Man erhält 105,6 g (60 % der Theorie) 5-Amino-1-(2,6-dichlor-4-trifluormethylphenyl)-3-tert.-butyl-pyrazol vom Schmelzpunkt 110 - 120 °C.

$$(CH_3)_3C-\underset{\overset{\|}{O}}{C}-CH_2CN$$

Zu einer Lösung aus 201,8 g (1,5 Mol) 1-Chlor-3,3-dimethyl-2-butanon und 800 ml Methanol tropft man bei Siedetemperatur eine Lösung aus 108,1 g (1,66 Mol) Kaliumcyanid in 270 ml Wasser und erhitzt 1 Stunde unter Rückfluß. Anschließend wird das Lösungsmitel abgezogen, der Rückstand in 1,5 l Eiswasser angeschlämmt, durch Zugabe von 66,4 g (1,5 Mol) Natriumhydroxid in Lösung gebracht und mit Ether gewaschen. Die wässrige Phase wird unter Eiskühlung angesäuert, mit Methylenchlorid extrahiert, über Magnesiumsulfat getrocknet und eingeengt.

Nach Umkristallisation aus Essigester/Ligroin erhält man 139,6 g (74,5 % der Theorie) 1-Cyan-3,3-dimethyl-2-butanon vom Schmelzpunkt 67 - 69 °C.

Beispiel 2

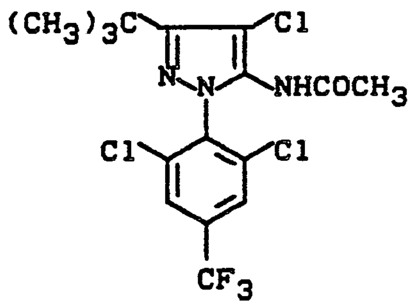

(I-2)

(Verfahren a)

Zu 23,6 g (0,06 Mol) 3-tert.-Butyl-5-acetamido-1-(2,6-dichlor-4-trifluormethylphenyl)-pyrazol in 250 ml absolutem Methylenchlorid werden bei Raumtemperatur 8,9 g (0,066 Mol) Sulfurylchlorid in 20 ml absolutem Methylenchlorid getropft. Man läßt über Nacht weiterrühren, engt dann die Reaktionsmischung auf die Hälfte des Volumens ein und saugt das ausgefallene Produkt ab.

Nach dem Trocknen erhält man 22,5 g (87,5 % der Theorie) 3-tert.-Butyl-4-chlor-5-acetamido-1-(2,6-dichlor-4-trifluormethylphenyl)-pyrazol vom Schmelzpunkt 211 °C.

Beispiel 3

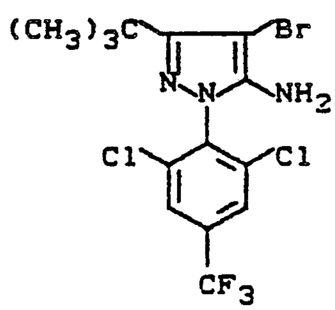

(I-3)

(Verfahren a)

Zu 17,6 g (0,05 Mol) 3-tert.-Butyl-5-amino-1-(2,6-dichlor-4-trifluormethylphenyl)-pyrazol in 50 ml absolutem Chloroform werden bei Raumtemperatur 8 g (0,05 Mol) Brom, gelöst in 20 ml absolutem Chloroform, getropft. Man läßt bei Raumtemperatur einige Stunden nachrühren, engt die Reaktionsmischung ein und kristallisiert den Rückstand aus wenig Methanol um.

Man erhält 20,9 g (97 % der Theorie) 3-tert.-Butyl-4-brom-5-amino-1-(2,6-dichlor-4-trifluormethylphenyl)-pyrazol vom Schmelzpunkt 228 - 231 °C.

Beispiel 4

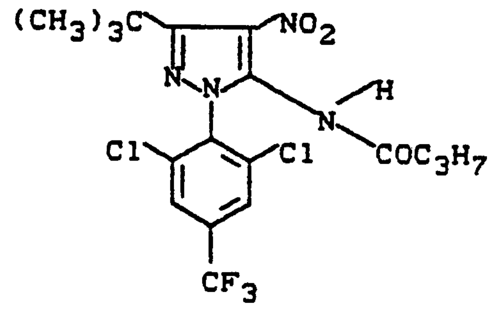

(I-4)

(Verfahren c - α)

4 g (0,01 Mol) 5-Amino-3-tert.-butyl-4-nitro-1-(2,6-dichlor-4-trifluormethylphenyl)-pyrazol, 1,6 g (0,015 Mol) Buttersäurechlorid, 2 Tropfen konz. Schwefelsäure und 40 ml Buttersäure werden 2 Stunden bei Raumtemperatur und weitere 12 Stunden bei 40 °C gerührt. Nach beendeter Reaktion versetzt man mit Methylenchlorid und wäscht die organische Phase 2 mal mit 2 N Natriumhydroxidlösung, 1 mal mit Wasser, trocknet über Magnesiumsulfat und entfernt das Lösungsmittel im Vakuum.

Nach Verreiben mit Petrolether erhält man 1,5 g (32 % der Theorie) 3-tert.-Butyl-5-butyramido-4-nitro-1-(2,6-dichlor-4-trifluormethylphenyl)-pyrazol vom Schmelzpunkt 152 - 154 °C.

Beispiel 5

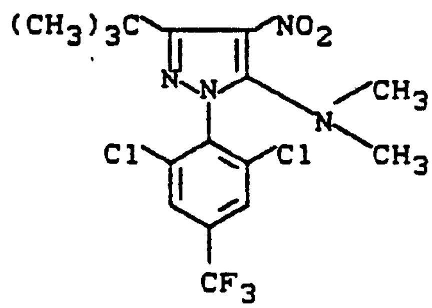

(I-5)

(Verfahren c - β)

7,9 g (0,01 Mol) 5-Amino-3-tert.-butyl-4-nitro-1-(2,6-dichlor-4-trifluormethylphenyl)-pyrazol werden in 160 ml Methylenchlorid gelöst und nacheinander mit 40 ml 45-%iger wässriger Natronlauge, einer Spatelspitze Tributylbenzylammoniumchlorid und 7,6 g (0,06 Mol) Dimethylsulfat versetzt und 16 Stunden bei Raumtemperatur gerührt. Die wässrige Phase wird abgetrennt, die organische mit Wasser gewaschen und im Vakuum vom Lösungsmittel befreit. Der Rückstand wird in 200 ml Ethanol aufgenommen, mit 20 ml 25%-igem wässrigen Ammoniak versetzt und 6 Stunden gerührt. Danach wird das Lösungsmittel im Vakuum entfernt, der Rückstand in 80 ml Methylenchlorid gelöst und die organische Phase nacheinander mit wässriger Ammoniumchlorid-und Kochsalzlösung gewaschen, über Magnesiumsulfat getrocknet und im Vakuum eingeengt.

Man erhält 6,9 g (81 % der Theorie) 3-tert.-Butyl-5-dimethylamino-4-nitro-1-(2,6-dichlor-4-trifluormethylphenyl)-pyrazol vom Schmelzpunkt 81 -83 °C.

Beispiel 6

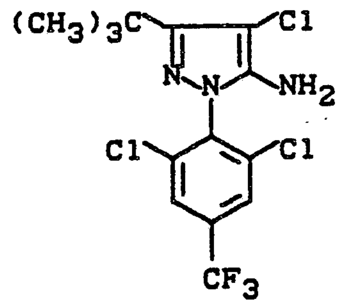

(I-6)

(Verfahren d)

52,8 g (0,12 Mol) 3-tert.-Butyl-4-chlor-5-acetamido-1-(2,6-dichlor-4-trifluormethylphenyl)-pyrazol werden in 250 ml Ethanol und 60 ml konzentrierter Salzsäure 8 Stunden unter Rückfluß erhitzt. Man engt die Reaktionsmischung ein, nimmt in Methylenchlorid auf und stellt mit 10-%iger Natriumhydroxidlösung alkalisch. Die organische Phase wird mit gesättigter Natriumchloridlösung gewaschen, über Magnesiumsulfat getrocknet, eingeengt und mit Petrolether verrieben.

Man erhält 43,2 g (93 % der Theorie) 3-tert.-Butyl-4-chlor-5-amino-1-(2,6-dichlor-4-trifluormethylphenyl)-pyrazol vom Schmelzpunkt 137 °C.

Beispiel 7

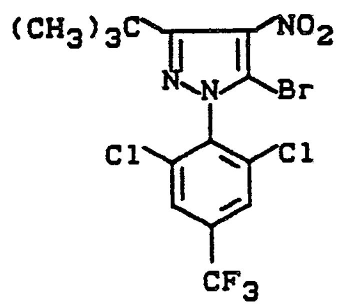

(I-7)

(Verfahren e)

Zu 7,9 g (0,02 Mol) 3-tert.-Butyl-4-nitro-5-amino-1-(2,6-dichlor-4-trifluormethylphenyl)-pyrazol in 50 ml Bromoform werden bei Raumtemperatur 3,6 ml (0,03 Mol) tert.-Butyl-nitrit gegeben. Die Temperatur steigt dabei auf 40 - 50 °C an. Die Reaktionsmischung wird noch einige Stunden nachgerührt und anschließend eingeengt, zum Schluß im Hochvaku um. Der Rückstand wird säulenchromatographisch über Kieselgel mit dem Laufmittel Petrolether : Essigester (9 : 1) gereinigt.

Man erhält 4,9 g (53 % der Theorie) 3-tert.-Butyl-4-nitro-5-brom-1-(2,6-dichlor-4-trifluormethylphenyl)-pyrazol vom Schmelzpunkt 105 - 108 °C.

Beispiel 8

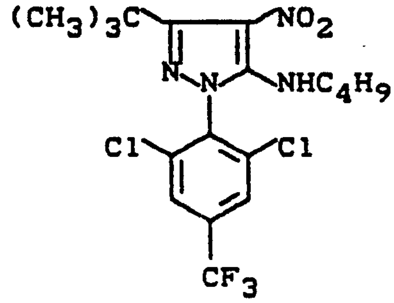

(I-8)

(Verfahren b)

6 g (0,013 Mol) 3-tert.-Butyl-4-nitro-5-brom-1-(2,6-dichlor-4-trifluormethylphenyl)-pyrazol werden in 10 ml n-Butylamin eingetragen. Die Reaktionsmischung wird erst 4 Stunden bei Raumtemperatur und anschließend 16 Stunden bei 40 °C nachgerührt. Nach beendeter Reaktion wird die Reaktionslösung in 200 ml Wasser gegeben und mit Methylenchlorid extrahiert. Die organische Phase wird mit 1 N Salzsäure und mit Wasser gewaschen, getrocknet und eingeengt. Der Rückstand wird säulenchromatographisch über Kieselgel mit dem Laufmittel Petrolether : Essigester (9 : 1) gereinigt.

Man erhält 3,6 g (61 % der Theorie) 3-tert.-Butyl-5-n-butylamino-4-nitro-1-(2,6-dichlor-4-trifluormethylphenyl)-pyrazol vom Schmelzpunkt 84 - 85 °C.

In entsprechender Weise und gemäß den allgemeinen Angaben zur Herstellung erhält man die folgenden substituierten 1-Aryl-3-tert.-butyl-pyrazole der allgemeinen Formel (I):

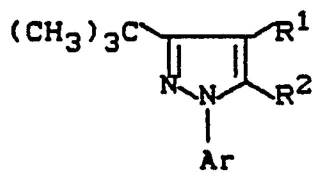

(I)

| Bsp. Nr. | $R^1$ | $R^2$ | Ar | Physikalische Daten |
|---|---|---|---|---|
| I-9 | $NO_2$ | $-NH_2$ | 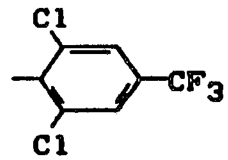 | Fp.: 168 - 71 °C |
| I-10 | Cl | H | 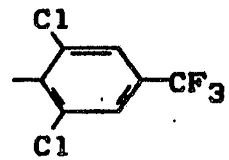 | $^1H$-NMR (in $CDCl_3$) t-Bu: 1,46 ppm (9H, s) 5-Pyraz.-H: 7,5 ppm (1H, s) |
| I-11 | Cl | $-N(CH_3)_2$ | 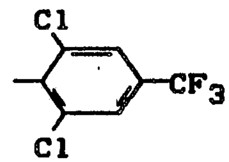 | Fp.: 79 - 80 °C |
| I-12 | $NO_2$ | Cl | 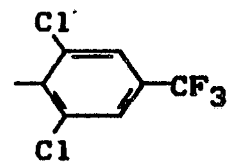 | Fp.: 114 °C |
| I-13 | Br | H | 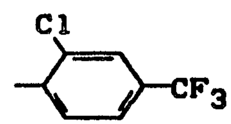 | $n_D^{23}$ : 1,5340 |
| I-14 | Br | H | 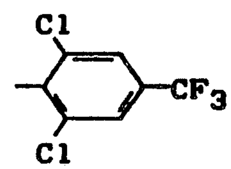 | $n_D^{23}$ : 1,5314 |

| Bsp. Nr. | $R^1$ | $R^2$ | Ar | Physikalische Daten |
|---|---|---|---|---|
| I-15 | Cl | H | 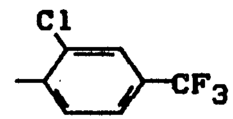 | $n_D^{23}$ : 1,5195 |
| I-16 | Cl | H | 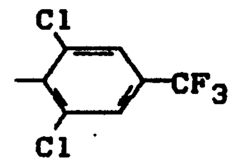 | Fp. : 89 - 90 °C |
| I-17 | Br | Cl | 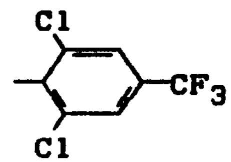 | Fp. : 88 - 89 °C |
| I-18 | Br | $NH_2$ | 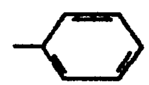 | $n_D^{20}$: 1,5908 |
| I-19 | Br | $NH_2$ | 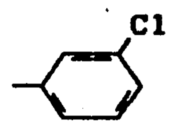 | $n_D^{20}$: 1,6008 |
| I-20 | Br | $NH_2$ | 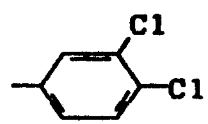 | Fp: 71° -73° C |

| Bsp. Nr. | $R^1$ | $R^2$ | Ar | Physikalische Daten |
|---|---|---|---|---|
| I-21 | Br | $NH_2$ | 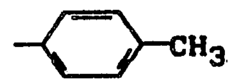 | Fp: 35° -40° C |
| I-22 | Br | $NH_2$ | 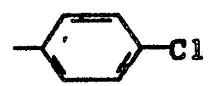 | Fp: 84° -86° C |
| I-23 | Br | $NH_2$ | 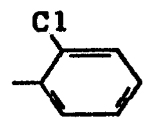 | Fp: 79° -83° C |
| I-24 | Br | H | 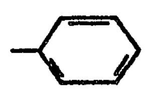 | $n_D^{20}$: 1,5778 |
| I-25 | Br | H | 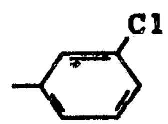 | $n_D^{20}$: 1,6043 |
| I-26 | Br | H | 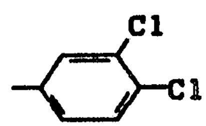 | $n_D^{20}$: 1,6158 |
| I-27 | Br | H | 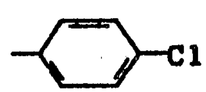 | $n_D^{20}$: 1,5913 |

| Bsp. Nr. | $R^1$ | $R^2$ | Ar | Physikalische Daten |
|---|---|---|---|---|
| I-28 | Br | H | 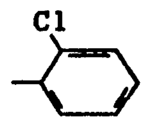 | Fp: 52° -53° C |
| I-29 | $NO_2$ | $NH_2$ | 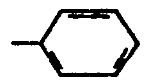 | Fp: 164° -165° C |
| I-30 | Cl | $NH_2$ | 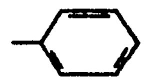 | $n_D^{20}$: 1,5749 |
| I-31 | Cl | $NH_2$ | 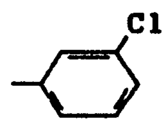 | $n_D^{20}$: 1,5836 |
| I-32 | Cl | $NH_2$ | 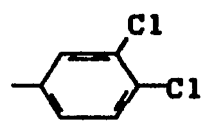 | Fp: 81° -82° C |
| I-33 | Cl | $NH_2$ | 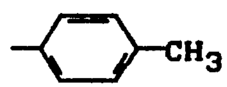 | Fp: 78° -80° C |

| Bsp. Nr. | $R^1$ | $R^2$ | Ar | Physikalische Daten |
|---|---|---|---|---|
| I-34 | Cl | $NH_2$ | 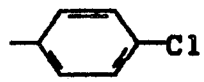 | Fp: 88° -90° C |
| I-35 | Cl | $NH_2$ | 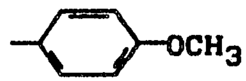 | Fp: 105° -106° C |
| I-36 | Cl | $NH_2$ | 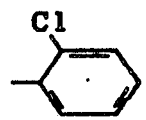 | Fp: 102° -103° C |
| I-37 | $NO_2$ | $NH_2$ | 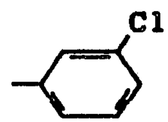 | Fp: 125° -126° C |
| I-38 | $NO_2$ | $NH_2$ | 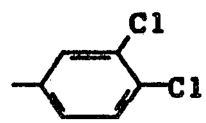 | Fp: 149° C |

| Bsp. Nr. | $R^1$ | $R^2$ | Ar | Physikalische Daten |
|---|---|---|---|---|
| I-39 | $NO_2$ | $NH_2$ | 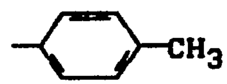 | Fp: 158°–160° C |
| I-40 | $NO_2$ | $NH_2$ | 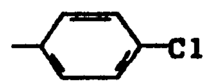 | Fp: 178° -179° C |
| I-41 | $NO_2$ | $NH_2$ | 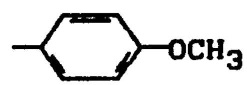 | Fp: 116° -117° C |
| I-42 | $NO_2$ | $NH_2$ | 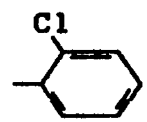 | Fp: 219° -220° C |

Herstellung weiterer Ausgangsstoffe der Formel (II)

Beispiel a

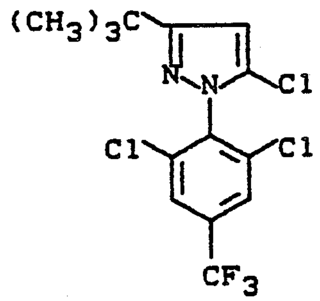

(II-3)

10 g (0,028 Mol) 1-(2,6-Dichlor-4-trifluormethylphenyl)-3-tert.-butyl-5-pyrazolon werden in 26,1 ml (0,28 Mol) Phosphoroxychlorid über Nacht im Bombenrohr unter Eigendruck auf 160 °C erhitzt. Anschließend wird vorsichtig auf Eiswasser ausgetragen und mit Methylenchlorid extrahiert. Die organische Phase wird mit 12prozentiger Ammoniaklösung und gesättigter Natriumchloridlösung gewaschen, über Magnesiumsulfat getrocknet und eingeengt.

Man erhält 8,3 g (80 % der Theorie) an 5-Chlor-1-(2,6-dichlor-4-trifluormethylphenyl)-3-tert.-butylpyrazol.

$^1$H-NMR (90 MHz/$CDCl_3$): $\delta$ = 1,33 (9 H, s, $C(CH_3)_3$); 6,31 (1 H, s, 4-Pyrazol-H); 7,72 (2 H, d, Aryl-H) ppm

Herstellung des Vorproduktes

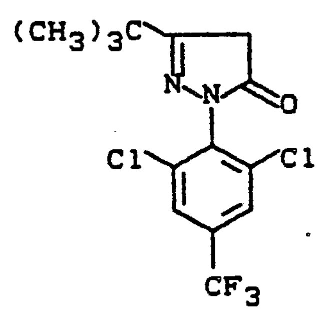

47.4 g (0,3 Mol) 4,4-Dimethyl-3-oxo-pentansäuremethylester und 73,5 g (0,3 Mol) 2,6-Dichlor-4-trifluormethylphenylhydrazin und 0,5 g p-Toluolsulfonsäure werden in 300 ml Toluol über Nacht am Wasserabscheider unter Rückfluß erhitzt. Anschließend wird das Lösungsmittel abdestilliert und der Rückstand mit Petrolether verrieben und abgesaugt.

Man erhält 96 g (91 % der Theorie) an 1-(2,6-Dichlor-4-trifluormethylphenyl)-3-tert.-butyl-5-pyrazolon vom Schmelzpunkt 175 °C.

Beispiel b

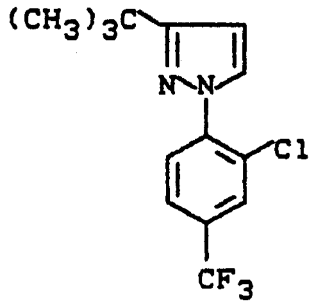

(II-4)

42 g (0,2 Mol) 2-Chlor-4-trifluormethylphenylhydrazin und 30 g (0,2 Mol) 1,1-Dimethyl-2-butanon-4,4-dimethylacetal werden in 400 ml Ethanol gelöst. Anschließend läßt man 4 Stunden bei 60 °C und weitere 6 Stunden unter Rückflußtemperatur rühren. Danach werden 4 ml konzentrierte Schwefelsäure hinzugegeben und über Nacht bei 60 °C nachgerührt. Nach beendeter Reaktion wird das Lösungsmittel abdestilliert und der Rückstand in Methylenchlorid aufgenommen, mit gesättigter Natriumhydrogencarbonatlösung und Wasser gewaschen, über Magnesiumsulfat getrocknet und eingeengt.

Man erhält 49 g (81 % der Theorie) 3-tert.-Butyl-1-(2-chlor-4-trifluormethylphenyl)-pyrazol vom Brechungsindex $n_D^{23}$ = 1,5070.

Entsprechend den Herstellungsbeispielen 1 sowie a und b und gemäß den allgemeinen Angaben zur Herstellung erhält man die folgenden Vorprodukte der Formel (II):

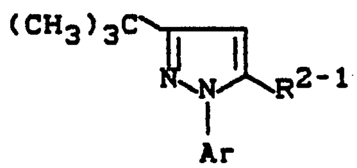

(II)

| Bsp. Nr. | $R^{2-1}$ | Ar | Physikalische Daten |
|---|---|---|---|
| II-5 | $-NHCOCH_3$ | 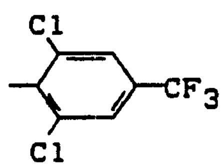 | Fp. : 165 -71 °C |

| Bsp. Nr. | $R^{2-1}$ | Ar | Physikalische Daten |
|---|---|---|---|
| II-6 | H | 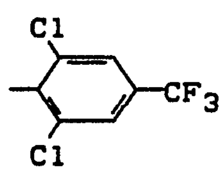 | $n_D^{20}$: 1,5082 |
| II-7 | $NH_2$ | 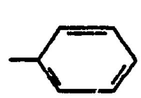 | Fp: 48° -50° C |

Anwendungsbeispiele

In den folgenden Anwendungsbeispielen wurden die nachstehend aufgeführten Verbindungen als Vergleichsverbindungen eingesetzt:

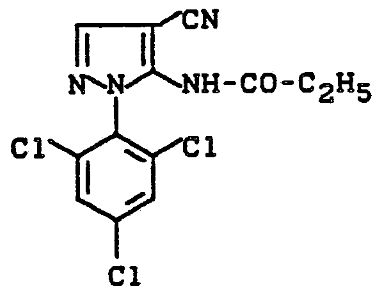

(A)

4-Cyano-5-propionylamino-1-(2,4,6-trichlorphenyl)-pyrazol (bekannt aus DE-OS 3 226 513)

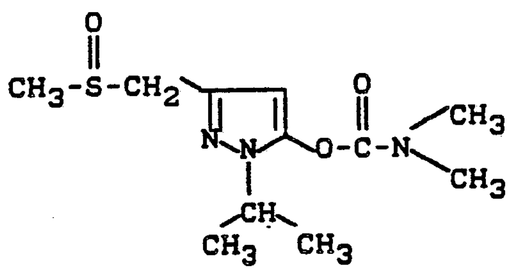

(B)

5-Dimethylaminocarbonyloxy-1-isopropyl-3-methylsulfinylmethylpyrazol (bekannt aus DE-OS 2 819 932)

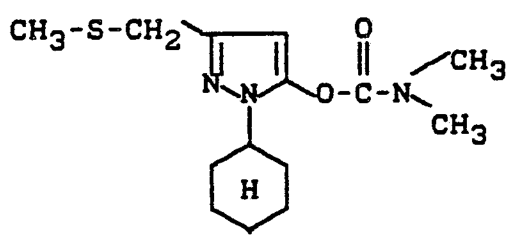

(C)

1-Cyclohexyl-5-dimethylaminocarbonyloxy-3-methylthiomethylpyrazol (bekannt aus DE-OS 2 839 270).

Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: I-6, I-3, I-7, I-9, I-1.

Beispiel B

Tetranychus-Test (resistent)
Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: I-7.

Beispiel C

$LT_{100}$-Test für Dipteren

Testtiere: Musca domestica
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirksamkeit gegenüber dem Stand der Technik: I-6, I-10.

Beispiel D

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Bei diesem Test zeigen z.B. die folgenden Verbindungen der Herstellungsbeipiele überlegene Wirksamkeit gegenüber dem Stand der Technik: I-9, I-6, I-10.

Beispiel E

$LD_{100}$-Test

Testtiere: Blatella germanica
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Bei diesem Test zeigen z.B. die folgenden Verbindungen der Herstellungsbeispiele überlegene Wirksamkeit gegenüber dem Stand der Technik: I-6, I-10, I-7.

Beispiel F

Test mit Stomoxys calcitrans

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: I-3.

Beispiel:

Facefly - Test (Musca autumnalis)

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

10 adulte Faceflies (Musca autumnalis) werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad in Prozent bestimmt, wobei 100% bedeuten, daß alle und 0%, daß keine Fliegen abgetötet worden sind.

Bei diesem Test zeigt z.B. die folgende Verbindung der Herstellungsbeispiele überlegene Wirkung gegenüber dem Stand der Technik: I-3.

## Ansprüche

1. 1-Aryl-3-tert.-butyl-pyrazole der allgemeinen Formel (I)

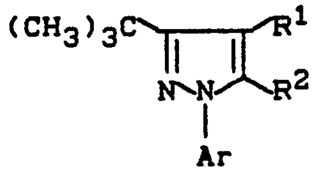

(I)

in welcher

$R^1$ für Nitro oder Halogen steht,

$R^2$ für Halogen oder die Gruppierung $-NR^3R^4$ steht, oder auch für Wasserstoff steht, wenn $R^1$ Halogen bedeutet, wobei

$R^3$ für Wasserstoff, für jeweils gegebenenfalls substituiertes Alkyl, Alkenyl oder Alkinyl oder für jeweils gegebenenfalls substituiertes Cycloalkyl oder Cycloalkylalkyl steht und

$R^4$ unabhängig von $R^3$ für die gleichen Reste wie $R^3$ steht, oder zusätzlich für einen Rest $-\underset{\underset{X}{\|}}{C}-R^5$ steht, wobei X für Sauerstoff oder Schwefel steht und

$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und

Ar für gegebenenfalls substituiertes Phenyl, ausgenommen Dinitrophenyl, oder für gegebenenfalls substituiertes Pyridyl steht.

2. 1-Aryl-3-tert.butyl-pyrazole der Formel (I) gemäß Anspruch 1, dadurch gekennzeichnet, daß

$R^1$ für Nitro, Fluor, Chlor, Brom oder Jod,

$R^2$ für Fluor, Chlor, Brom, Jod oder die Gruppierung $-NR^3R^4$; sowie auch für Wasserstoff steht, wenn $R^1$ für ein Halogen steht, wobei

$R^3$ für Wasserstoff oder für jeweils geradkettiges oder verzweigtes, gegebenenfalls einfach oder mehrfach, gleich oder verschieden substituiertes Alkyl, Alkenyl und Alkinyl mit jeweils bis zu 8 Kohlenstoffatomen steht, wobei als Substituenten jeweils infrage kommen: Halogen, Cyano, Nitro, Hydroxy, Mercapto, Carboxy, jeweils geradkettiges oder verzweigtes Alkoxy, Alkylthio und Alkoxycarbonyl mit jeweils bis zu 6 Kohlenstoffatomen; ferner für jeweils gegebenenfalls einfach oder mehrfach, gleich oder verschieden substituiertes Cycloalkyl mit 3 bis 7 Kohlenstoffatomen und Cycloalkylalkyl mit 3 bis 7 Kohlenstoffatomen im Cycloalkylteil und 1 bis 4 Kohlenstoffatomen im Alkylteil steht, wobei als Substituenten im Cycloalkylteil jeweils in Frage kommen: Halogen und geradkettiges oder verzweigtes Alkyl mit 1 bis 4 Kohlenstoffatomen; und

$R^4$ unabhängig von $R^3$ für die gleichen Reste wie $R^3$ steht, sowie zusätzlich für einen Rest $-\underset{\underset{X}{\|}}{C}-R^5$ steht, wobei

X für Sauerstoff oder Schwefel steht und

$R^5$ für Wasserstoff, für geradkettiges oder verzweigtes Alkyl mit 1 bis 12 Kohlenstoffatomen, für jeweils geradkettiges oder verzweigtes Alkenyl oder Alkinyl, mit jeweils 2 bis 4 Kohlenstoffatomen, für jeweils geradkettiges oder verzweigtes Alkoxyalkyl, Alkylthioalkyl, Alkoxy, Alkylthio, Alkylamino, Dialkylamino oder Halogenalkyl mit jeweils 1 bis 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen; sowie

Ar für jeweils gegebenenfalls einfach oder mehrfach gleich oder verschieden substituiertes Phenyl (ausgenommen Dinitrophenyl), 2-Pyridyl, 3-Pyri dyl oder 4-Pyridyl steht, wobei als Substituenten jeweils infrage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^6$, wobei
$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
m für eine Zahl 0, 1 oder 2 steht.

3. 1-Aryl-3-tert.butyl-pyrazole der allgemeinen Formel (I) gemäß Anspruch 1, dadurch gekennzeichnet, daß
$R^1$ für Nitro, Chlor oder Brom steht:
$R^2$ für Chlor, Brom oder die Gruppierung $-NR^3R^4$ steht; sowie auch für Wasserstoff steht, wenn $R^1$ für ein Halogen steht, wobei
$R^3$ für jeweils gegebenenfalls ein-bis dreifach, gleich oder verschieden substituiertes Methyl, Ethyl, n-oder i-Propyl, n-, i-, s-oder t-Butyl, Allyl, Butenyl, Propargyl oder Butinyl steht, wobei als Substituenten infrage kommen: Fluor, Chlor, Brom, Iod, Cyano, Nitro, Hydroxy, Mercapto, Carboxy, Methoxy, Ethoxy, n-oder i-Propoxy, Methylthio, Ethylthio, n-oder i-Propylthio, Methoxycarbonyl, Ethoxycarbonyl, n-oder i-Propoxycarbonyl, n-, i-, s-oder t-Butoxycarbonyl; ferner für jeweils gegebenenfalls einfach bis dreifach, gleich oder verschieden im Cycloalkylteil durch Chlor, Methyl oder Ethyl substituiertes Cyclopropyl, Cyclopropylmethyl, Cyclopentyl, Cyclopentylmethyl, Cyclohexyl, Cyclohexylmethyl, Cyclohexylethyl, Cycloheptyl oder Cycloheptylmethyl;
$R^4$ unabhängig von $R^3$ für die gleichen Reste wie $R^3$ steht, sowie zusätzlich für einen Rest $-\underset{\underset{X}{\|}}{C}-R^5$
steht, wobei
X für Sauerstoff oder Schwefel steht und
$R^5$ für Wasserstoff, Methyl, Ethyl, n-oder i-Propyl, n-, i-, s-oder t-Butyl, Undecyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein-bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht; sowie
Ar für gegebenenfalls ein-bis fünffach, gleich oder verschieden substituiertes Phenyl (ausgenommen Dinitrophenyl) oder für jeweils gegebenenfalls ein-bis vierfach, gleich oder verschieden substituiertes 2-Pyridyl, 3-Pyridyl oder 4-Pyridyl steht, wobei als Substituenten jeweils infrage kommen: Cyano, Nitro, Fluor, Chlor, Brom, Iod, Methyl, Ethyl, n-oder i-Propyl, n-, i-, s-oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^6$, wobei
$R^6$ für Amino, Methylamino, Ethylamin, Dimethylamino, Diethylamino, Fluordichlormethyl, Difluormethyl, Tetrafluorethyl, Trichlorethyl, Trifluormethyl, Difluorchlormethyl, Methyl oder Ethyl steht und
m für eine Zahl 0, 1 oder 2 steht.

4. Verfahren zur Herstellung von 1-Aryl-3-tert.butyl-pyrazolen der allgemeinen Formel

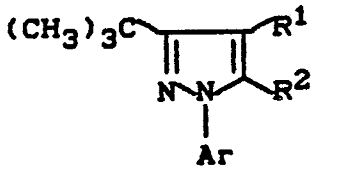

(I)

in welcher
$R^1$ für Nitro oder Halogen steht,
$R^2$ für Halogen oder die Gruppierung $-NR^3R^4$ steht, oder auch für Wasserstoff steht, wenn $R^1$ Halogen bedeutet, wobei
$R^3$ für Wasserstoff, für jeweils gegebenenfalls substituiertes Alkyl, Alkenyl oder Alkinyl oder für jeweils

gegebenenfalls substituiertes Cycloalkyl oder Cycloalkylalkyl steht und
$R^4$ unabhängig von $R^3$ für die gleichen Reste wie $R^3$ steht, oder zusätzlich für einen Rest $-\underset{\underset{X}{\|}}{C}-R^5$ steht, wobei
X für Sauerstoff oder Schwefel steht und
$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und
Ar für gegebenenfalls substituiertes Phenyl, ausgenommen Dinitrophenyl, oder für gegebenenfalls substituiertes Pyridyl steht,
dadurch gekennzeichnet, daß man
a) zum Erhalt von substituierten 1-Aryl-3-tert.butyl-pyrazolen der Formel (I),

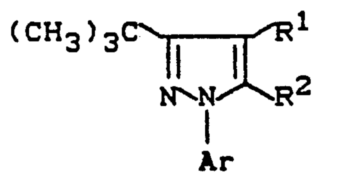
(I)

in welcher
$R^1$, $R^2$ und Ar die oben angegebene Bedeutung haben,
1-Aryl-3-tert.-butyl-pyrazole der Formel (II),

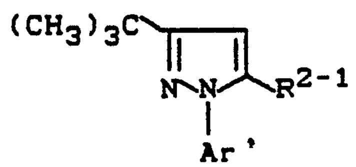
(II)

in welcher
$R^{2-1}$ für Wasserstoff, Halogen oder die Gruppierung $-NR^3R^4$ steht, wobei
$R^3$ und $R^4$ die oben angegebene Bedeutung haben und
Ar die oben angegebene Bedeutung hat, mit Halogenierungs-oder Nitrierungsmitteln der Formel (III),
$R^1$ -A (III)
in welcher
$R^1$ die oben angegebene Bedeutung hat und
A für eine elektronenanziehende Abgangsgruppe steht,
gegebenenfalls in Gegenwart eines Verdünnungsmittels und gegebenenfalls in Gegenwart eines Katalysators oder Reaktionshilfsmittels umsetzt;
b) oder daß man zum Erhalt von substituierten 1-Aryl-3-tert.-butyl-pyrazolen der Formel (Ia),

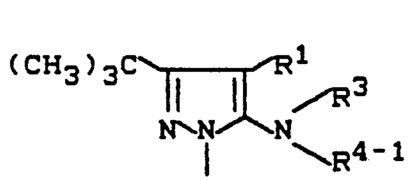
(Ia)

in welcher
$R^1$, $R^3$ und Ar die oben angegebene Bedeutung haben und
$R^{4-1}$ unabhängig von $R^3$ für die gleichen Reste wie $R^3$ steht,
die nach Verfahren (a) erhältlichen 1-Aryl-5-halogen-3-tert.-butyl-pyrazole der Formel (Ib),

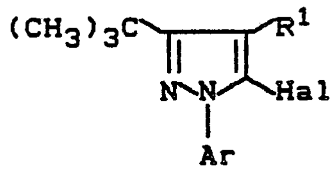

(Ib)

in welcher
$R^1$ und Ar die oben angegebene Bedeutung haben und
Hal für Halogen, vorzugsweise für Brom oder Chlor steht,
mit Aminen der Formel (IV),

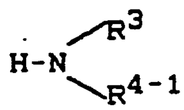

(IV)

in welcher
$R^3$ und $R^{4-1}$ die oben angegebene Bedeutung haben,
gegebenenfalls in Gegenwart eines Verdünnungsmittels und gegebenenfalls in Gegenwart eines Säurebindemittels umsetzt;
c) oder daß man zum Erhalt von substituierten 1-Aryl-3-tert.-butyl-pyrazolen der Formel (Ic),

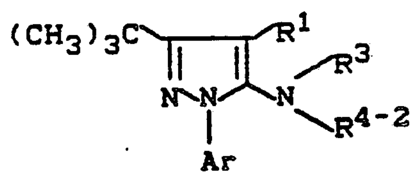

(Ic)

in welcher
$R^1$, $R^3$ und Ar die oben angegebene Bedeutung haben und
$R^{4-1}$ für die oben angegebenen Bedeutungen von $R^4$, ausgenommen Wasserstoff, steht,
die nach den Verfahren (a) und (b) erhältlichen 5-Amino-1-aryl-3-tert.-butyl-pyrazole der Formel (Id),

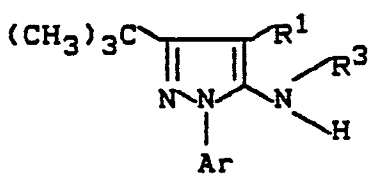

(Id)

in welcher
$R^1$, $R^3$ und Ar die oben angegebene Bedeutung haben,
α) mit Acylierungsmitteln der Formel (V),

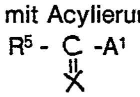

(V)

in welcher
$R^5$ und X die oben angegebene Bedeutung haben und
$A^1$ für Halogen oder den Rest $R^5$-CO-O-steht, wobei
$R^5$ die oben angegebene Bedeutung hat, oder
β) mit Alkylierungsmitteln der Formel (VI),
$R^{4-2}$ -$A^2$ (VI)
in welcher
$R^{4-2}$ die oben angegebene Bedeutung hat und
$A^2$ für eine elektronenanziehende Abgangsgruppe steht,
jeweils gegebenenfalls in Gegenwart eines Verdünnungsmittels und gegebenenfalls in Gegenwart eines Säurebindemittels sowie gegebenenfalls in Gegenwart eines Katalysators umsetzt;

d) oder daß man zum Erhalt von 5-Amino-1-aryl-3-tert.-butyl-pyrazolen der Formel (Id),

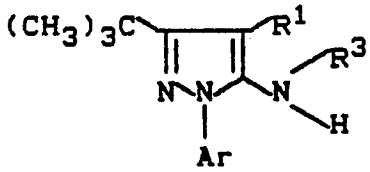

(Id)

in welcher
$R^1$, $R^3$ und Ar die oben angegebene Bedeutung haben,
die nach den Verfahren (a) oder (c) erhältlichen 1-Aryl-3-tert.-butyl-pyrazole der Formel (Ie),

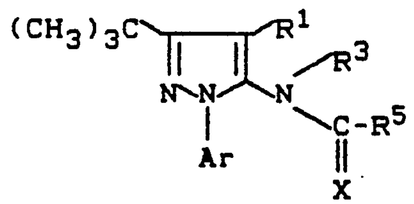

(Ie)

in welcher
$R^1$, $R^3$, $R^5$, X und Ar die oben angegebene Bedeutung haben,
gegebenenfalls in Gegenwart eines Verdünnungsmittels und gegebenenfalls in Gegenwart eines Katalysators deacyliert;
e) oder daß man zum Erhalt von 1-Aryl-3-tert.-butyl-pyrazolen der Formel (If),

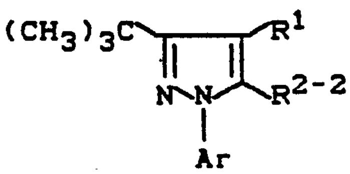

(If)

in welcher
$R^1$ und Ar die oben angegebene Bedeutung haben und
$R^{2-2}$ für Halogen steht, sowie auch für Wasserstoff steht, wenn $R^1$ Halogen bedeutet,
die nach den Verfahren (a), (b) oder (d) erhältlichen 5-Amino-1-aryl-3-tert.-butyl-pyrazole der Formel (Ig),

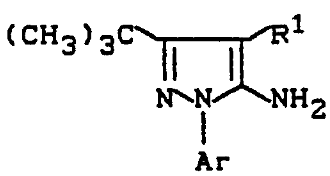

(Ig)

in welcher
$R^1$ und Ar die oben angegebene Bedeutung haben,
mit einem anorganischen oder organischen Nitrit in Gegenwart eines Reaktionshilfsmittels und gegebenenfalls in Gegenwart einer Halogenwasserstoffsäure sowie gegebenenfalls in Gegenwart eines Verdünnungsmittels umsetzt.

5. Schädlingsbekämpfungsmittel, gekennzeichnet durch einen Gehalt an mindestens einem 1-Aryl-3-tert.butyl-pyrazol der Formel (I).

6. Insektizide und akarizide Mittel, gekennzeichnet durch einen Gehalt an mindestens einem 1-Aryl-3-tert.butyl-pyrazol der Formel (I).

7. Verfahren zur Bekämpfung von Insekten und Spinnentieren, dadurch gekennzeichnet, daß man 1-Aryl-3-tert.butyl-pyrazole der Formel (I) auf Insekten und/oder Spinnentiere und/oder deren Lebensraum einwirken läßt.

8. Verwendung von 1-Aryl-3-tert.butyl-pyrazolen der Formel (I) zur Bekämpfung von Insekten und/oder Spinnentieren.

9. Verfahren zur Herstellung von Schädlingsbekämpfungsmitteln, dadurch gekennzeichnet, daß man 1-Aryl-3-tert.butyl-pyrazole der Formel (I) mit Streckmitteln und/oder oberflächenaktiven Mitteln vermischt.

10. 1-Aryl-3-tert.-butyl-pyrazole der Formel (II),

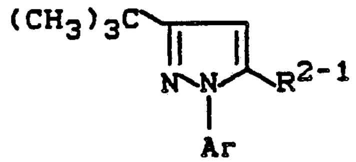

(II)

in welcher
$R^{2-1}$ für Wasserstoff, Halogen oder die Gruppierung $-NR^3R^4$ steht, wobei
$R^3$ für Wasserstoff, für jeweils gegebenenfalls substituiertes Alkyl, Alkenyl oder Alkinyl sowie für jeweils gegebenenfalls substituiertes Cycloalkyl oder Cycloalkylalkyl steht und
$R^4$ unabhängig von $R^3$ für die gleichen Reste wie $R^3$ steht, sowie zusätzlich für einen Rest $-\underset{\overset{\|}{X}}{C}-R^5$ steht, wobei
X für Sauerstoff oder Schwefel steht und
$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und
Ar für gegebenenfalls substituiertes Phenyl, ausgenommen Dinitrophenyl, oder für gegebenenfalls substituiertes Pyridyl steht.

11. Verfahren zur Herstellung von 1-Aryl-3-tert.-butyl-pyrazole der Formel (II),

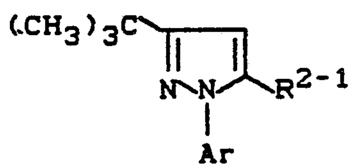

(II)

in welcher
$R^{2-1}$ für Wasserstoff, Halogen oder die Gruppierung $-NR^3R^4$ steht, wobei
$R^3$ für Wasserstoff, für jeweils gegebenenfalls substituiertes Alkyl, Alkenyl oder Alkinyl sowie für jeweils gegebenenfalls substituiertes Cycloalkyl oder Cycloalkylalkyl steht und
$R^4$ unabhängig von $R^3$ für die gleichen Reste wie $R^3$ steht, sowie zusätzlich für einen Rest $-\underset{\overset{\|}{X}}{C}-R^5$ steht, wobei
X für Sauerstoff oder Schwefel steht und
$R^5$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, für Dialkylamino oder für gegebenenfalls substituiertes Arylamino steht; und
Ar für gegebenenfalls substituiertes Phenyl, ausgenommen Dinitrophenyl, oder für gegebenenfalls substituiertes Pyridyl steht,
dadurch gekennzeichnet, daß man zum Erhalt von 1-Aryl-3-tert.butyl-pyrazolen der Formel (IIa)

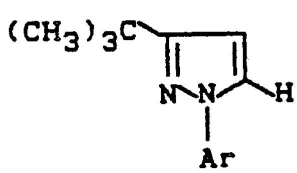

(IIa)

in welcher
Ar die oben angegebene Bedeutung hat,
(2,2-Dimethoxy)-ethyl-tert.-butyl-keton der Formel (VII),
$(CH_3)_3C-CO-CH_2CH(OCH_3)_2$ (VII)
mit Hydrazinen der Formel (VIII),
$Ar-NH-NH_2$ (VIII)
in welcher
Ar die oben angegebene Bedeutung hat,
gegebenenfalls in Gegenwart eines Verdünnungsmittels bei Temperaturen zwischen -50 °C und + 150 °C cyclisiert,
oder daß man zum Erhalt von 1-Aryl-5-halogen-3-tert.butyl-pyrazolen der Formel (IIb),

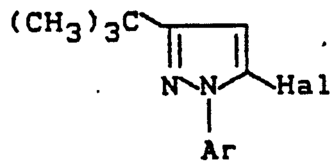
(IIb)

in welcher
Ar und Hal die oben angegebene Bedeutung haben, Pyrazolin-5-one der Formel (IX),

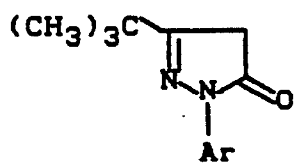
(IX)

in welcher
Ar die oben angegebene Bedeutung hat,
mit Phosphoroxyhalogeniden bei Temperaturen zwischen 100 und 250 °C umsetzt,
oder daß man zum Erhalt von 5-Amino-1-aryl-3-tert.butyl-pyrazolen der Formel (IIc)

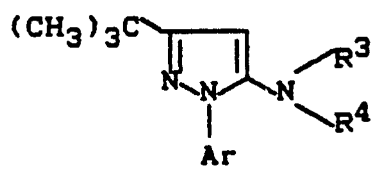
(IIc)

in welcher
Ar, $R^3$ und $R^4$ die oben angegebene Bedeutung haben,
5-Amino-1-aryl-3-tert.-butyl-pyrazole der Formel (IIe),

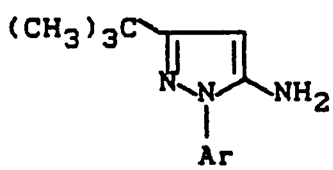
(IIe)

in welcher
Ar die oben angegebene Bedeutung hat,
α) mit Acylierungsmitteln der Formel (V),

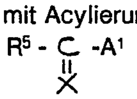
(V)

in welcher

$R^5$ und X die oben angegebene Bedeutung haben und
$A^1$ für Halogen oder den Rest $R^5$-CO-O-steht, wobei
$R^5$ die oben angegebene Bedeutung hat, oder
$\beta$) mit Alkylierungsmitteln der Formel (VI),

$R^{4-2}$ -$A^2$ (VI)

in welcher
$R^{4-2}$ die oben angegebene Bedeutung hat und
$A^2$ für eine elektronenanziehende Abgangsgruppe steht,
jeweils gegebenenfalls in Gegenwart eines Verdünnungsmittels und gegebenenfalls in Gegenwart eines Säurebindemittels sowie gegebenenfalls in Gegenwart eines Katalysators umsetzt.

Europäisches Patentamt

# EUROPÄISCHER RECHERCHENBERICHT

Nummer der Anmeldung

EP 87111791.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,A | EP - A2 - 0 201 852 (BAYER AG)<br>* Zusammenfassung *<br>-- | 1,4-11 | C 07 D 231/16<br>C 07 D 401/04<br>A 01 N 43/56 |
| P,A | DE - A1 - 3 623 302 (BAYER AG)<br>* Zusammenfassung *<br>-- | 1,4-11 | |
| P,A | CHEMICAL ABSTRACTS, Band 106, Nr. 21, 25. Mai 1987, Columbus, Ohio, USA<br>TAKEDA CHEMICAL INDUSTRIES "Nitrogen-containing heterocyclic compounds as microbicides" Seite 263, Spalte 2, Zusammenfassung-Nr. 171 125f<br>& Jpn. Kokai Tokkyo Koho JP 62 00 404 (87 00 404), 06 Jan. 1987<br>---- | 1,4-11 | RECHERCHIERTE SACHGEBIETE (Int. Cl.4)<br>C 07 D 231/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 19-11-1987 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82